# EUROPEAN PATENT APPLICATION

(11) **EP 4 818 165 A1**
(43) Date of publication of application: **07.10.2026**
(21) Application number: 24899913.8
(22) Date of filing: 05.12.2024
(51) Int. Cl.: C07D 401/14, C07D 413/14, A61K 31/444, A61P 35/00, A61P 35/02, A61P 37/02, A61P 25/00, A61P 31/00

(54) **IKZF1 DEGRADATION AGENT AND PREPARATION METHOD THEREFOR, AND APPLICATION**

(30) Priority: 05.12.2023 CN 202311652658
(71) Applicant: Guangzhou Yufan Nantu Biotechnologies Co., Ltd, Nansha District, Guangzhou 511453 (CN)
(72) Inventor: LIN, Xingyu, Zhuhai, Guangdong 519031 (CN); AN, Na, Zhuhai, Guangdong 519031 (CN); LU, Tingting, Zhuhai, Guangdong 519031 (CN); CHAI, Yushuang, Zhuhai, Guangdong 519031 (CN); LIU, Yahui, Zhuhai, Guangdong 519031 (CN)
(74) Representative: Viering, Jentschura & Partner mbB Patent- und Rechtsanwälte
(86) International application number: PCT/CN2024/137115
(87) International publication number: WO 2025/119279

(57) **Abstract**

The present disclosure provides an IKZF1 degrader, and a preparation method and use thereof, especially in preventing and/or treating IKZF1-related diseases. The degrader has the structure as defined in claim 1, and exhibits excellent targeting property against IKZF1.

## Description

### CROSS-REFERENCE TO RELATED APPLICATION

The application claims the priority of the Chinese Patent application filed on December 5, 2023 before the China National Intellectual Property Administration (CNIPA), with the application number of 202311652658.6, and the title of "IKZF1 DEGRADER, AND PREPARATION METHOD AND USE THEREOF", which is incorporated herein in its entirety by reference.

### FIELD

The present disclosure relates to the technical field of chemical medicine, and in particular, to an IKZF1 degrader, and a preparation method and use thereof, especially in preventing and/or treating IKZF1-related diseases.

### BACKGROUND

The Ikaros family is a series of zinc finger protein transcription factors that are important for particular physiological processes, especially for lymphocyte development. Ikaros (IKZF1) was first discovered in 1992, and over the subsequent two decades, four additional homologues have been identified: Helios (IKZF2), Aiolos (IKZF3), Eos (IKZF4), and Pegasus (IKZF5). Each of these homologous genes can produce several protein isoforms through alternative splicing. Theoretically, a large number of protein complexes are allowed to be formed through different combinations of various homologues. The distribution of different members of the Ikaros family varies considerably in vivo. IKZF1, IKZF2, and IKZF3 are primarily found in lymphoid cells and their corresponding progenitor cells. Additionally, IKZF1 has been detected in the brain, and both IKZF1 and IKZF2 have been detected in erythrocytes. IKZF4 and IKZF5 are more widely distributed and are found in skeletal muscle, liver, brain, and heart. IKZF1 is important for the normal development of lymphocytes. Deletion of the exons encoding the first three N-terminal zinc fingers results in mice lacking T cells, B cells, natural killer (NK) cells, and their progenitors. Genetic alterations of IKZF1 are related to adverse outcomes in the treatment of acute lymphoblastic leukemia (ALL). IKZF1 and IKZF3 are involved in the proliferation of multiple myeloma cells, indicating their potential roles in hematological malignancies.

Mutations in the IKZF1 gene have also been found to be related to the occurrence, development, proliferation, metastasis, and prognosis of various other malignancies (such as breast carcinoma, ovarian carcinoma, liver carcinoma, skin carcinoma, lung carcinoma, and colorectal carcinoma). They are also related to the complex phenotype and susceptibility of systemic lupus erythematosus (SLE).

Molecular glue degraders are a class of small molecules that can induce interactions between an E3 ubiquitin ligase substrate receptor and a target protein, thereby leading to degradation of the target protein. Currently, there are few reports on IKZF1 degraders.

### SUMMARY

To overcome the shortcomings of the prior art, the present disclosure provides an IKZF1 degrader, and a preparation method and use thereof. The degrader exhibits excellent targeting property against IKZF1.

In a first aspect of the present disclosure, there is provided a compound having the following structure: 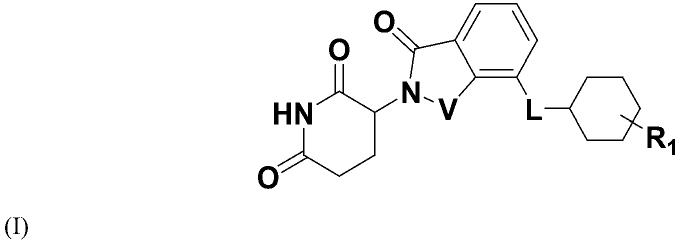 wherein
V is selected from the group consisting of: -CH₂-, 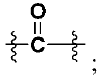
L has a structure of: 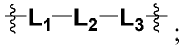
L₁ is selected from the group consisting of: -NH-(C₀₋₁₀ alkylene)-, -O-(C₀₋₁₀ alkylene)-;
L₂ is selected from the group consisting of: 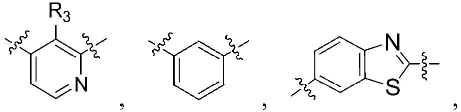 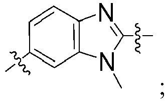 R₃ is selected from the group consisting of: -H, halogen, -CN, -NO₂, -CF₃, -CHF₂, -CH₂F, -OH, -OCH₃, -OCF₃, -OCHF₂, -OCH₂F, C₁₋₃ alkyl;
L₃ is selected from the group consisting of: -(C₀₋₁₀ alkylene)-, -(C₀₋₆ alkylene)-NH-(C₀₋₆ alkylene)-, -(C₀₋₆ alkylene)-O-(C₀₋₆ alkylene)-;
R₁ is one or more independent substituents on a ring 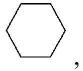 and is selected from the group consisting of: =O, halogen, -CN, -NO₂, -CF₃, -OCF₃, C₀₋₁₀ alkyl, -O(C₀₋₁₀ alkyl), -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -SC₀₋₁₀ alky, -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), 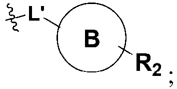 L' is selected from the group consisting of: single bond, -(C₀₋₁₀ alkylene)-, -(C₀₋₁₀ alkylene)-NR^{L1}-( C₀₋₁₀ alkylene)-, -(C₀₋₁₀ alkylene)-O-(C₀₋₁₀ alkylene)-, -(C₀₋₁₀ alkylene)-S-(C₀₋₁₀ alkylene)-, -(C₀₋₁₀ alkylene)-NR^{L1}CO-(C₀₋₁₀ alkylene)-, -(C₀₋₁₀ alkylene)-CONR^{L1}-(C₀₋₁₀ alkylene)-, -(C₀₋₁₀ alkylene)-CO-(C₀₋₁₀ alkylene)-, -(C₀₋₁₀ alkylene)-SO₂-(C₀₋₁₀ alkylene)-, -(C₀₋₁₀ alkylene)-SO-(C₀₋₁₀ alkylene)-; R^{L1} is selected from the group consisting of: **H,** C₁₋₆ alky, C₃₋₆ cycloalkyl, **4-10** membered heterocycloalkyl; R₂ is one or more independent substituents on a B ring, and is selected from the group consisting of: =O, halogen, -CN, -NO₂, -CF₃, -OCF₃, C₀₋₁₀ alkyl, -O(C₀₋₁₀ alkyl), -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -SC₀₋₁₀ alkyl, -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), -(C₀₋₁₀ alkylene)-(C₃₋₆ cycloalkyl), -(C₀₋₁₀ alkylene)-(C₆₋₁₀ aryl), -(C₀₋₁₀ **alkylene)-(4-10membered** heterocycloalkyl), -(C₀₋₁₀ **alkylene)-(4-10** membered heteroaryl), wherein one or more hydrogen atoms of the alkylene, alkyl, cycloalkyl, aryl, heterocycloalkyl, and heteroaryl are each independently optionally substituted with a group selected from: =O, halogen, -CN, -NO₂, -CF₃, -OCF₃, C₀₋₁₀ alkyl, -O(C₀₋₁₀ alkyl), -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -SC₀₋₁₀ alky, -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), - (C₀₋₁₀ alkylene)-(C₃₋₆ cycloalkyl), -(C₀₋₁₀ alkylene)-(C₆₋₁₀ aryl), -(C₀₋₁₀ alkylene)-(4-10membered heterocycloalkyl), -(C₀₋₁₀ **alkylene)-(4-10** membered heteroaryl).

In some embodiments of the present disclosure, V is

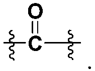

In some embodiments of the present disclosure,R₃is halogen, such as F.

In some embodiments of the present disclosure, L₁is -NH-, -NH-CH₂-, -NH-CH₂CH₂-, - NH-CH₂CH₂CH₂-, -O-, -O-CH₂-, -O-CH₂CH₂-, -O-CH₂CH₂CH₂-.

In some embodiments of the present disclosure, L₃ is -NH-, -CH₂-NH-, -CH₂CH₂-NH-, - CH₂CH₂CH₂-NH-, -O-, -CH₂-O-, -CH₂CH₂-O-, -CH₂CH₂CH₂-O-, single bond, -CH₂-, -CH₂CH₂-, -CH₂CH₂CH₂-, -NH-CH₂-, -NH-CH₂CH₂-, -NH-CH₂CH₂CH₂-, -O-CH₂-, -O-CH₂CH₂-, -O-CH₂CH₂CH₂-.

In some embodiments of the present disclosure, L has a structure of:

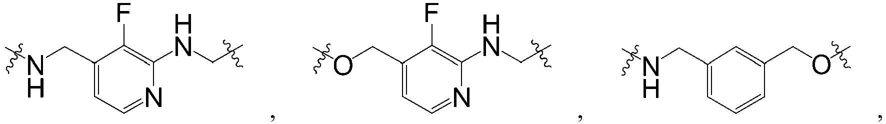

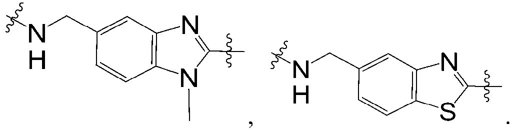

In some embodiments of the present disclosure, R₁ is one or more independent substituents on a ring 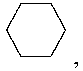 and is selected from the group consisting of: -H, a halogen, -CN, - NO₂, -CF₃, -OCF₃, C₁₋₁₀ alkyl, -O(C₀₋₁₀ alkyl), -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -SC₀₋₁₀ alkyl, -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), - CON(C₀₋₁₀ alkyl)(C₀₋₁₀ alkyl), and -CO(C₀₋₁₀ alkyl). More specifically, each R₁ is selected from the group consisting of: -H, a halogen (e.g., F), -OH, C₁₋₆ alkyl (e.g., -CH₃ or -CH₂CH₃), and - O(C₁₋₆ alkyl) (e.g., methoxy or ethoxy), and in particular -H.

In some embodiments of the present disclosure, a moiety 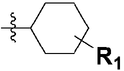 is 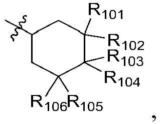 wherein R₁₀₁, R₁₀₂, R₁₀₃, R₁₀₄, R₁₀₅, and R₁₀₆ each have the definition as defined for R₁. Specifically, R₁₀₃, R₁₀₄, R₁₀₅, and R₁₀₆ may all be H (i.e., 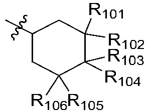 is 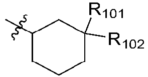 ). Specifically, R₁₀₁ and R₁₀₂ are independently selected from the group consisting of: -H, a halogen (e.g., F), -OH, C₁₋₆ alkyl (e.g., -CH₃ or -CH₂CH₃), and -O(C₁₋₆ alkyl) (e.g., methoxy or ethoxy). More specifically, 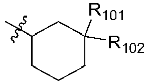 may be, for example,

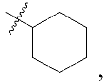

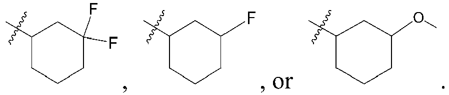

In some embodiments of the present disclosure, a moiety 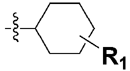 is 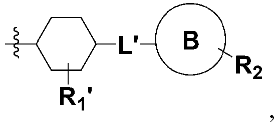 wherein R1' is one or more independent substituents on the 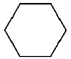 ring, selected from: H, halogen, -CN, -NO₂, -CF₃,-OCF₃, C₁₋₁₀ alkyl, -O(C₀₋₁₀ alkyl), -N(C₀₋₁₀ alkyl)(C₀₋₁₀ alkyl), -N(C₀₋₁₀ alkyl)C(O)(C₀₋₁₀ alkyl), -N(C₀₋₁₀ alkyl)C(O)N(C₀₋₁₀ alkyl), -N(C₀₋₁₀ alkyl)SO₂(C₀₋₁₀ alkyl), -S(C₀₋₁₀ alkyl), -S(O)(C₀₋₁₀ alkyl), -SO₂(C₀₋₁₉ alkyl), -SO₂N(C₀₋₁₀ alkyl)(C₀₋₁₀ alkyl), -C(O)O(C₀₋₁₀ alkyl),-OC(O)(C₀₋₁₀ alkyl), -C(O)N(C₀₋₁₀ alkyl)(C₀₋₁₀ alkyl), and -C(O)(C₀₋₁₀ alkyl); more specifically, each R1' is selected from: H, halogen, C₁₋₆ alkyl, and -O(C₀₋₆ alkyl), particularly H.

In some embodiments of the present disclosure, the compound may have the following structure:

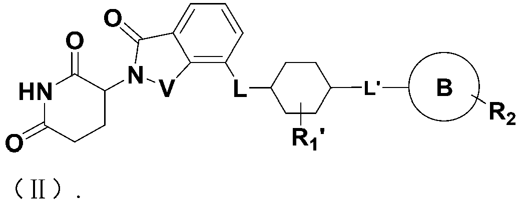

In some embodiments of the present disclosure, L' is single bond.

Specifically, ring B may be an aromatic ring or a heteroaromatic ring, such as a benzene ring, a bicyclic aromatic ring, a tricyclic aromatic ring, a monocyclic heteroaromatic ring, a bicyclic heteroaromatic ring, or a tricyclic heteroaromatic ring.

In some embodiments of the present disclosure, ring B is a benzene ring.

In some embodiments of the present disclosure, ring B is a monocyclic heteroaryl ring, such as

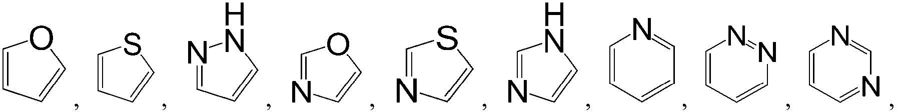

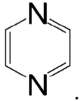

In some embodiments of the present disclosure, ring B is a bicyclic heteroaryl ring, such as a 5,5-fused or 6,5-fused heteroaryl ring. Specifically, it may be: 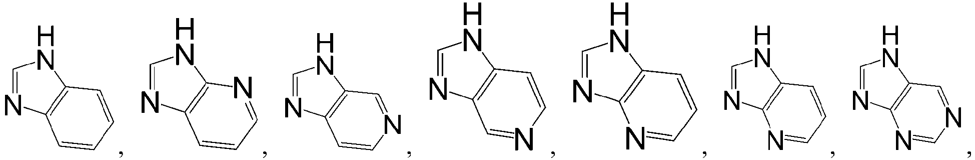 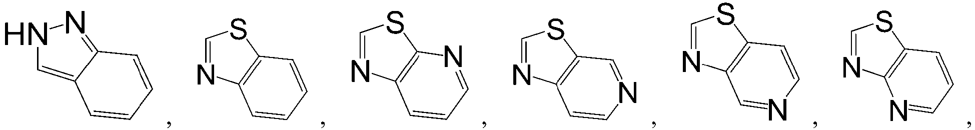 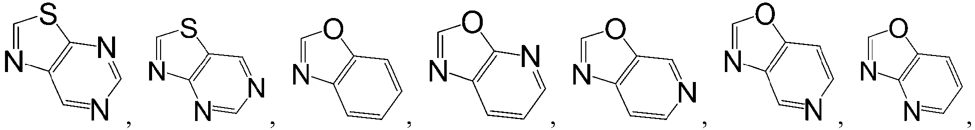 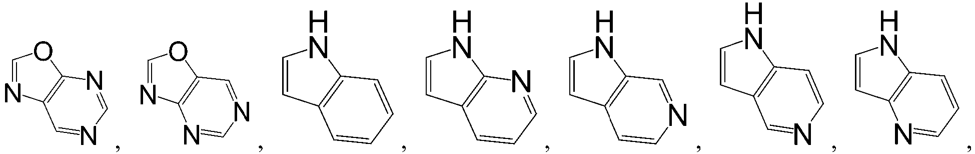 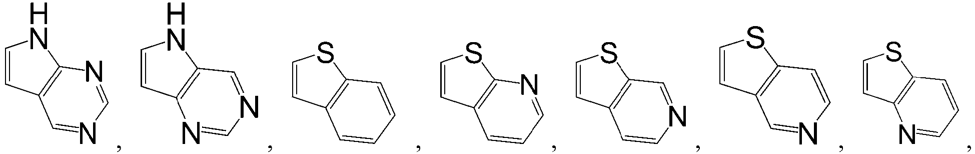 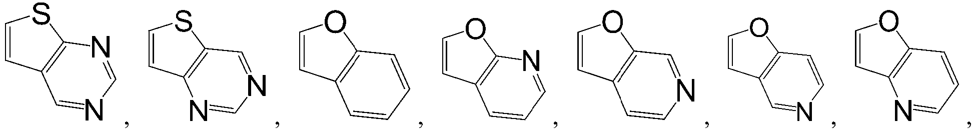 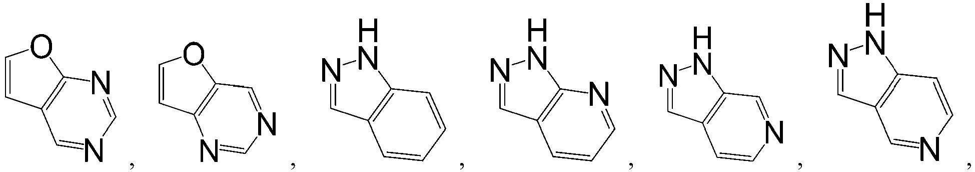 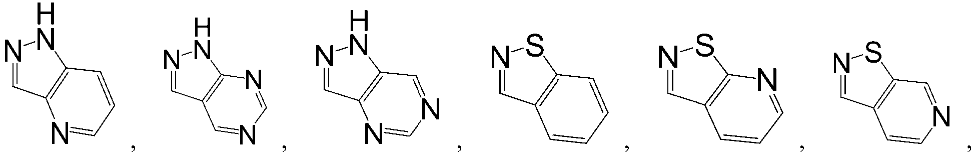 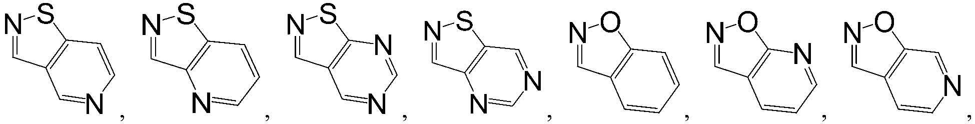 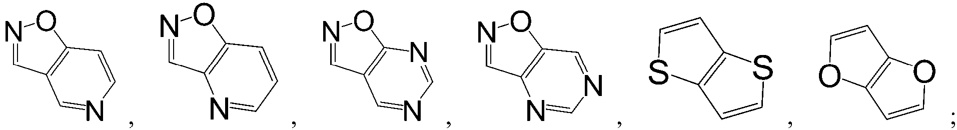 in particular,

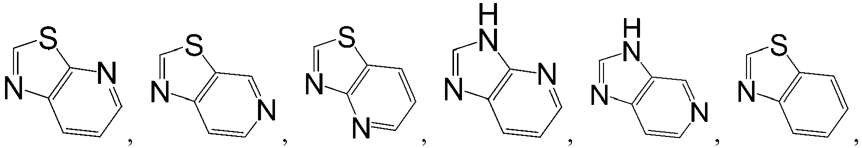

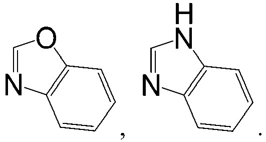

In some embodiments of the present disclosure, ring B is a tricyclic heteroaryl ring, such as a 5,6,5-fused, 6,5,5-fused, or 5,6,6-fused heteroaryl ring. Specifically, it may be:

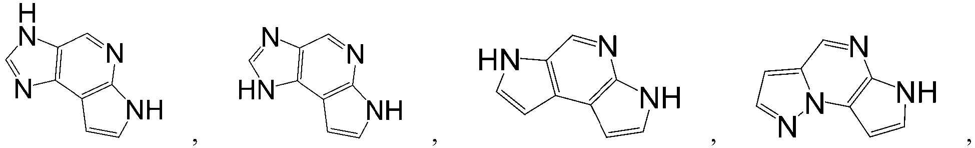

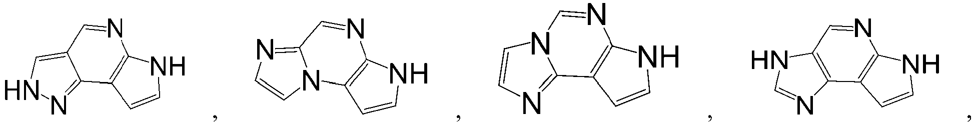

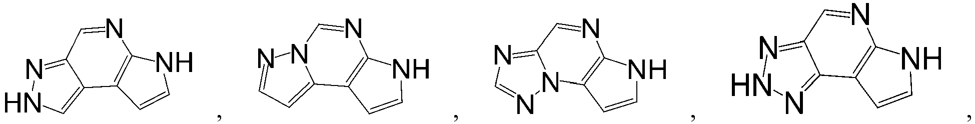

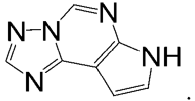

In some embodiments of the present disclosure, a moiety 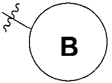 may have the following structure:

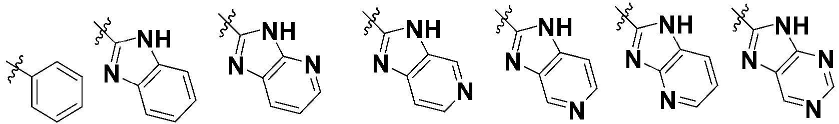

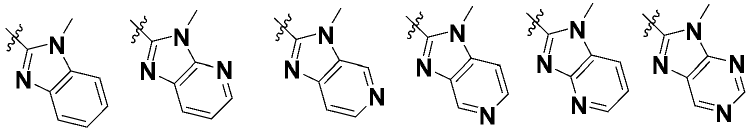

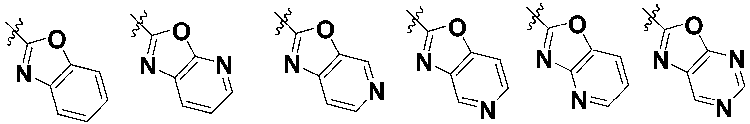

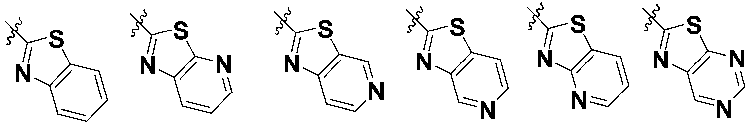

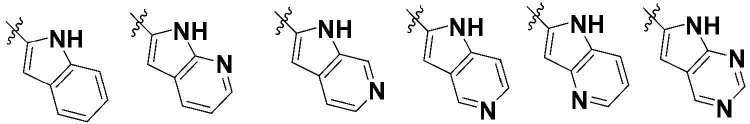

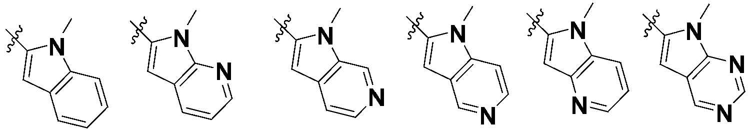

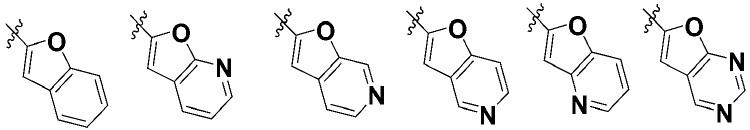

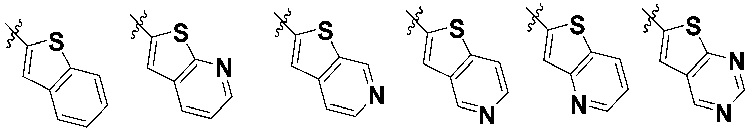

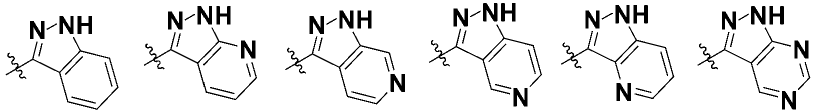

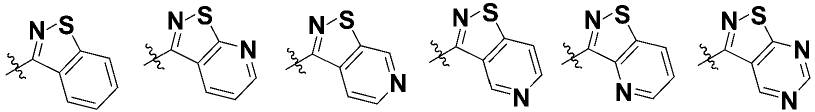

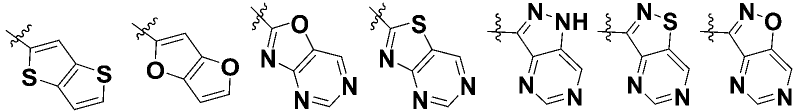

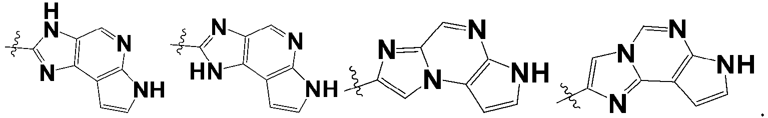

Specifically, R₂ may be selected from the group consisting of: -H, -F, -Cl, -Br, -I, -CF₃, - CHF₂, -CH₂F, -CH₂CF₃, -CN, -NO₂, -CH₃, 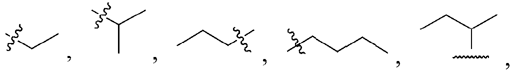 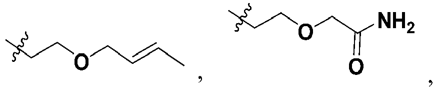 -OH, 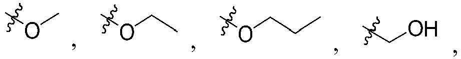 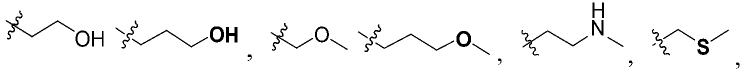 -NH₂,

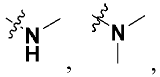

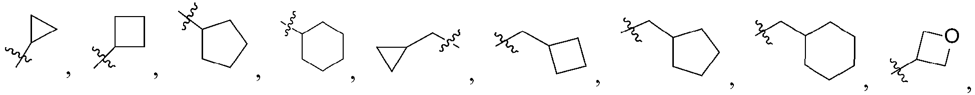

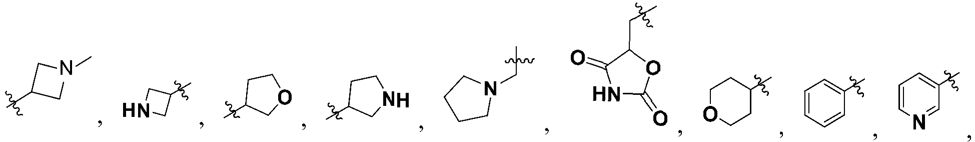

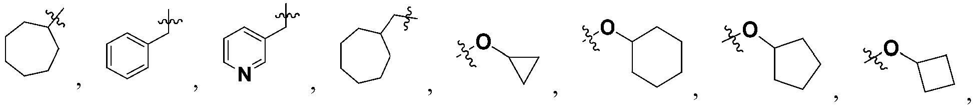

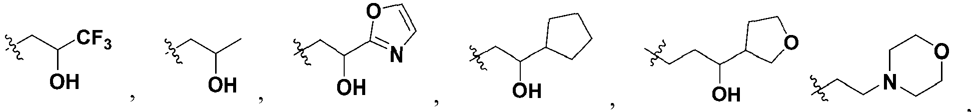

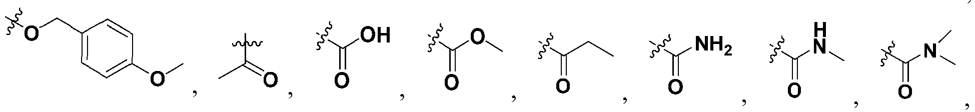

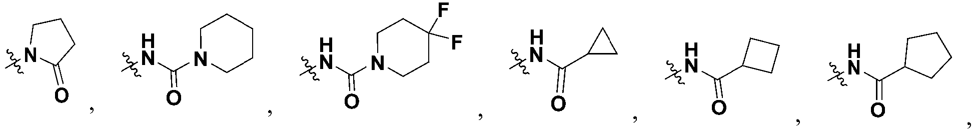

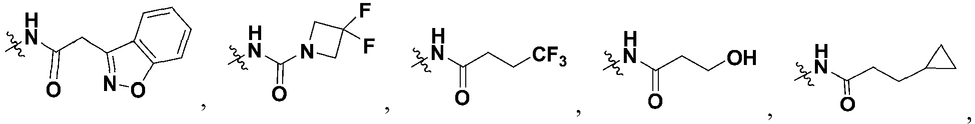

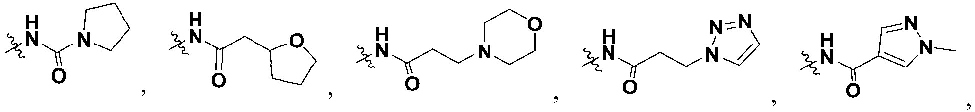

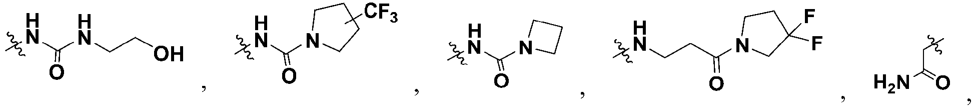

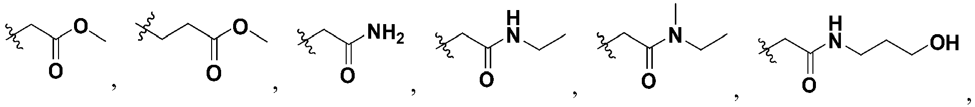

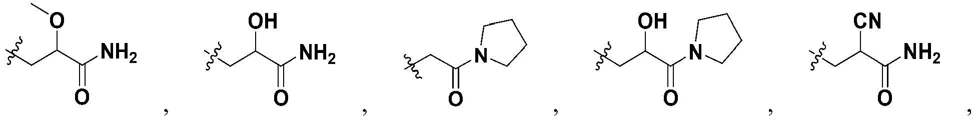

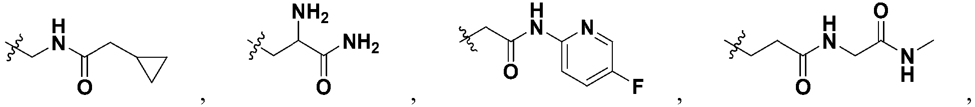

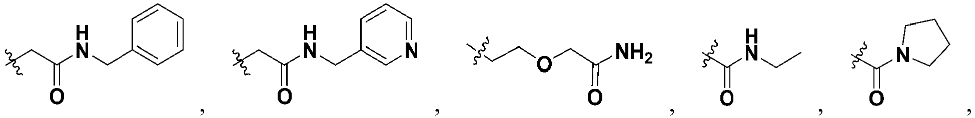

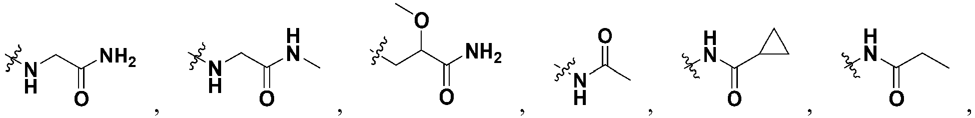

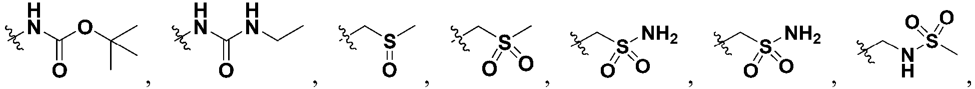

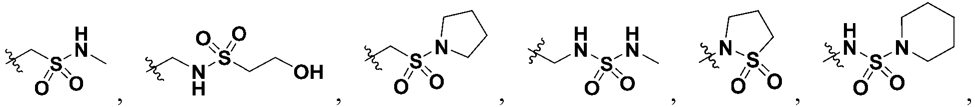

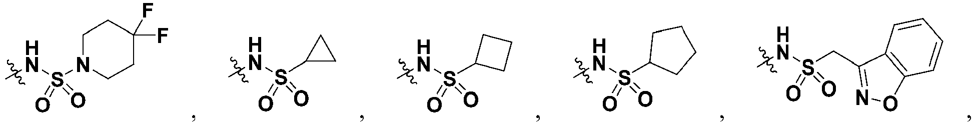

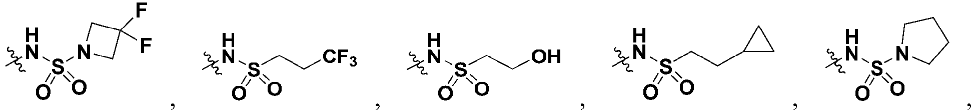

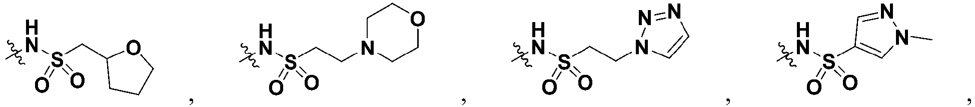

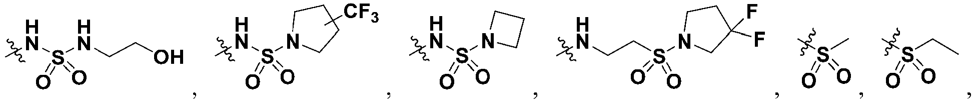

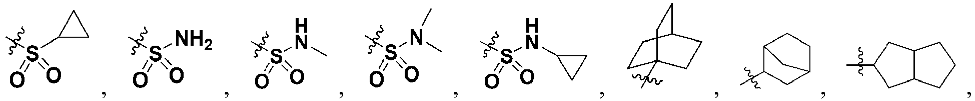

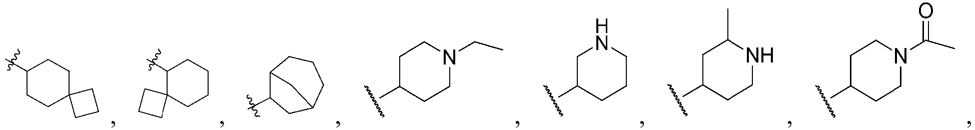

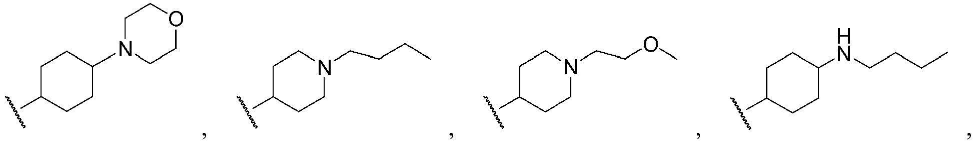

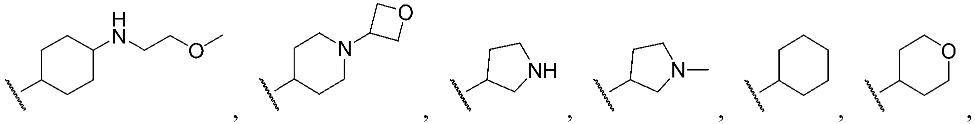

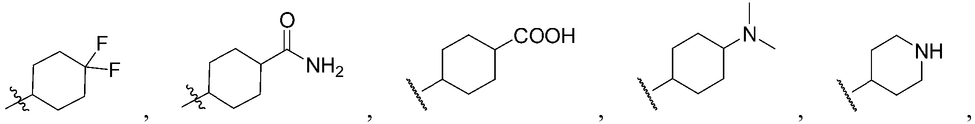

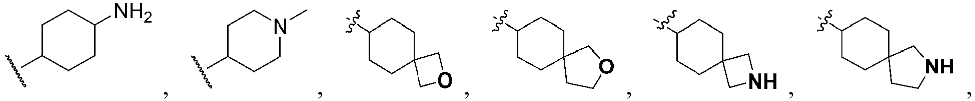

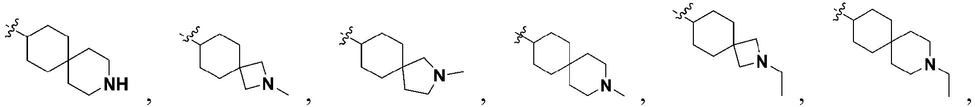

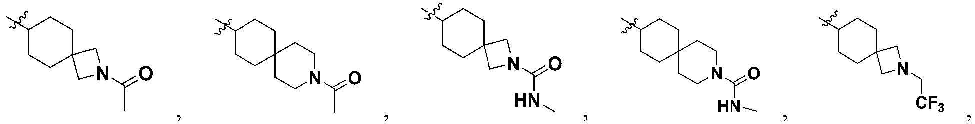

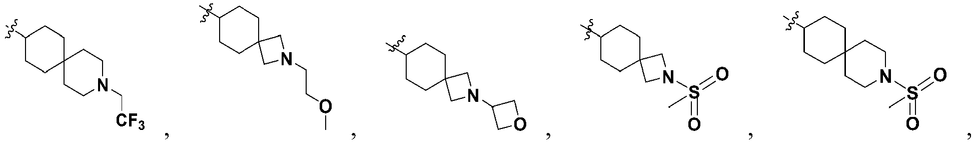

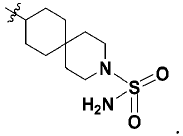

More specifically, R₂may be selected from the group consisting of: -H, -F, -Cl, -Br, -I, - CF₃, -CHF₂, -CH₂F, -CH₂CF₃, -CN, -NO₂, -CH₃, 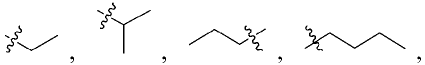 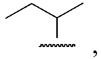 -OH, 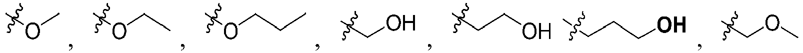 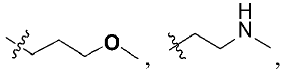 -NH₂,

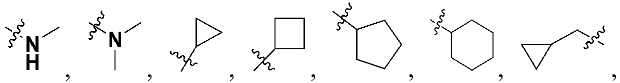

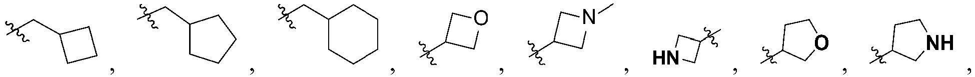

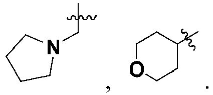

In some embodiments of the present disclosure, the compound may have the following structure: 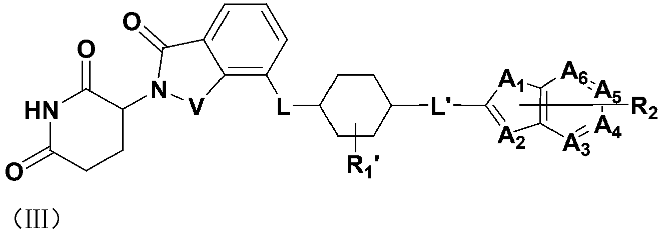
wherein, A₁ is selected from the group consisting of: O, S, NH, CH₂;
A₂ is selected from the group consisting of: N, CH;
A₃, A₄, A₅, A₆ are independently selected from: N, CH.

In some embodiments of the present disclosure, A₃, A₄, A₅ are CH.

In some embodiments of the present disclosure,A₆ is N.

In some embodiments of the present disclosure, A₂ is N.

In some embodiments of the present disclosure,A₁ is S.

In some embodiments of the present disclosure,A₁ is O.

In some embodiments of the present disclosure, A₁ is NH.

In some embodiments of the present disclosure, the compound has a structure of:

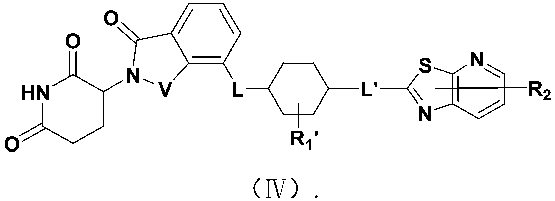

In some embodiments of the present disclosure, a moiety 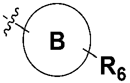 has a structure of:

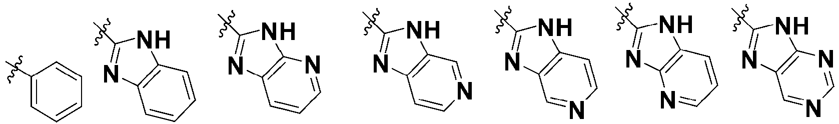

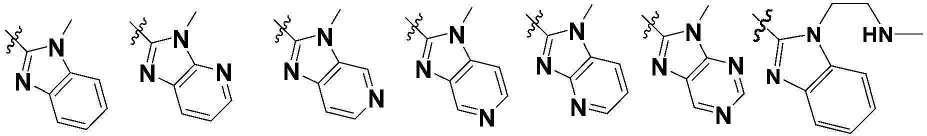

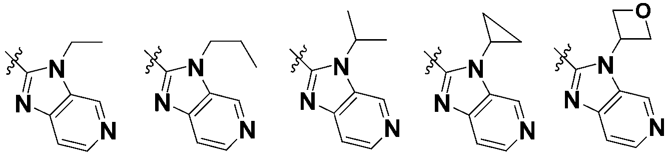

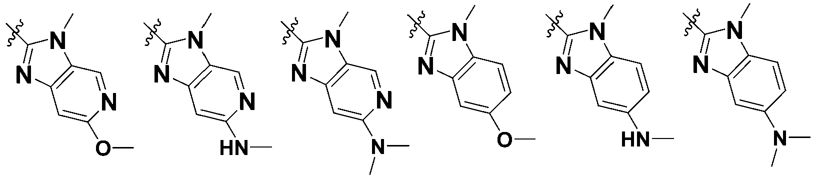

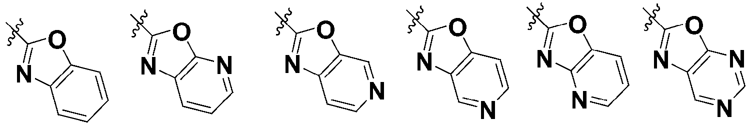

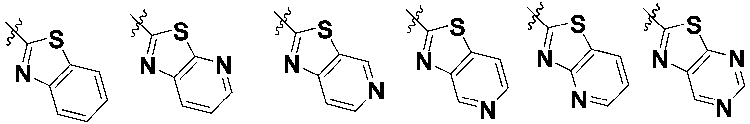

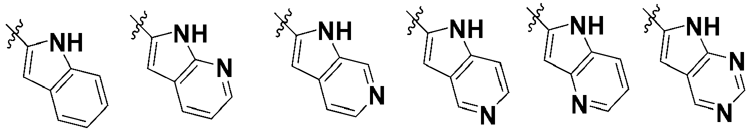

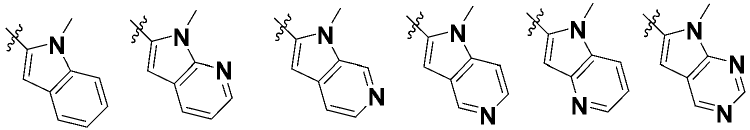

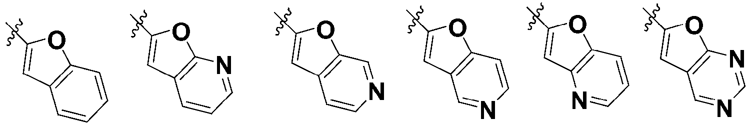

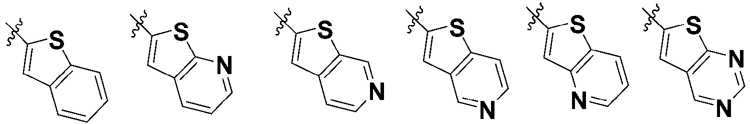

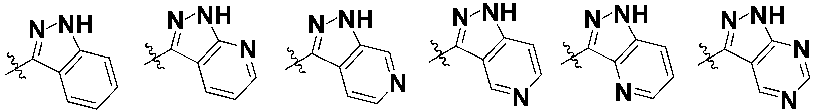

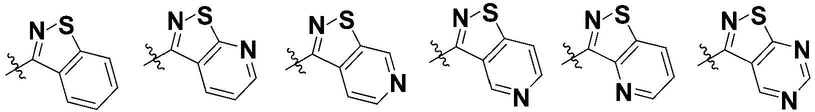

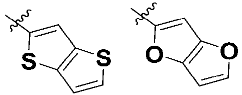

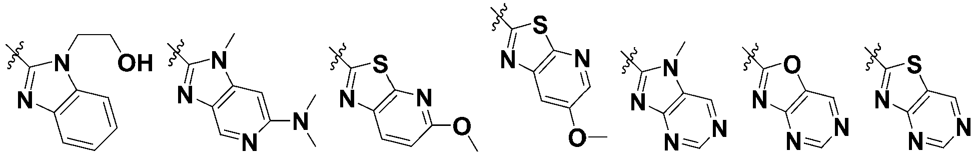

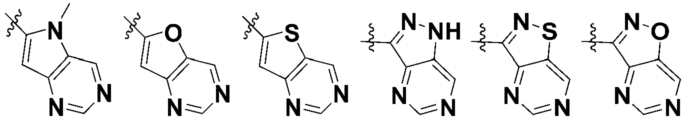

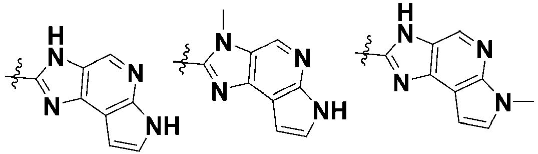

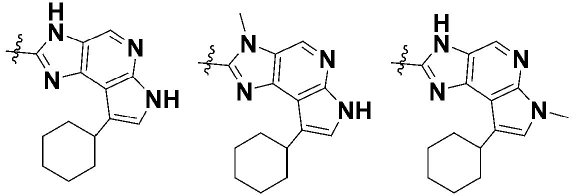

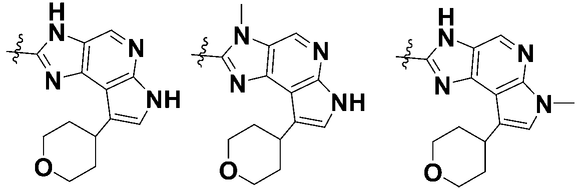

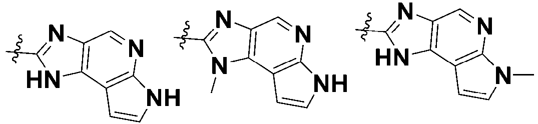

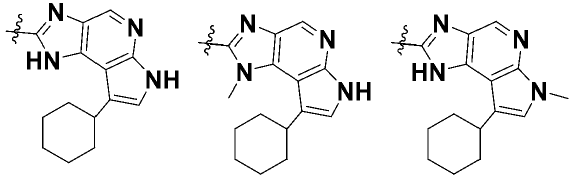

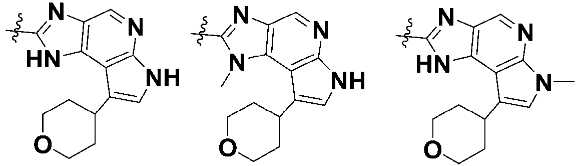

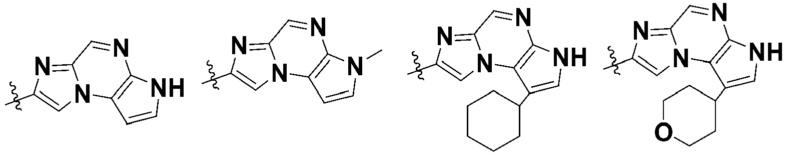

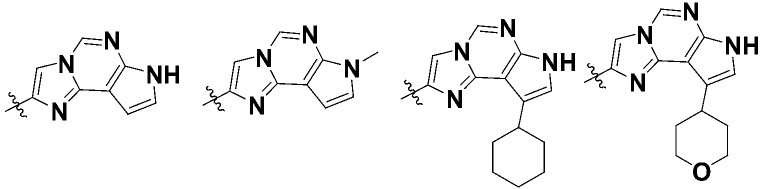

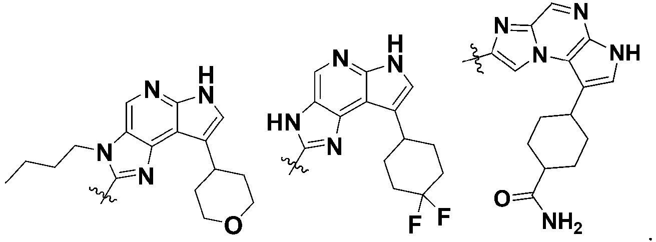

In some embodiments of the present disclosure, the compound has the structure of:

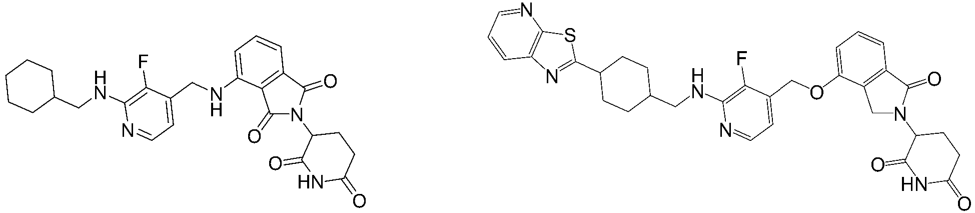

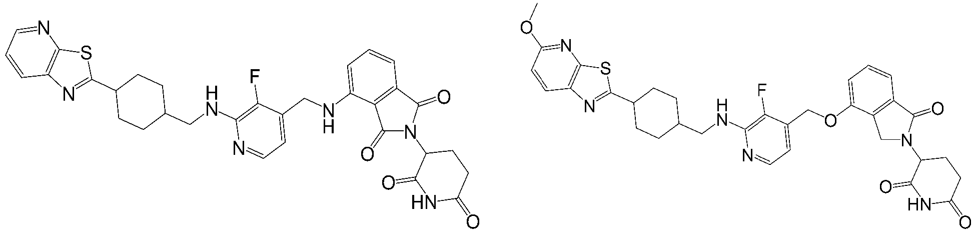

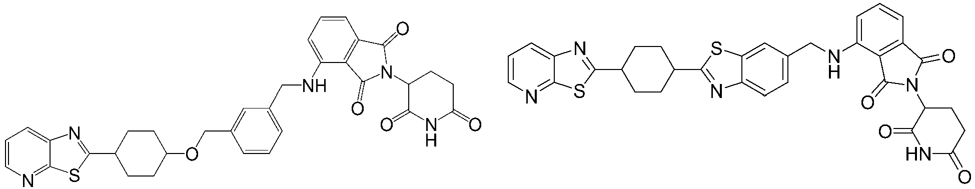

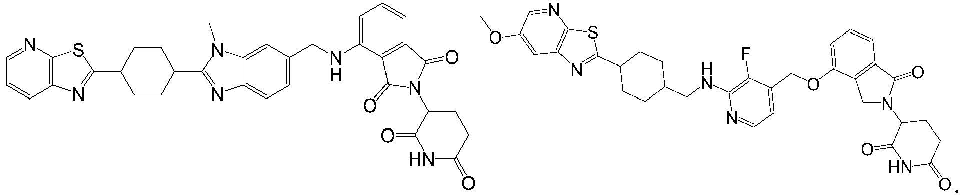

In a second aspect of the present disclosure, there is provided pharmaceutically acceptable salts, stereoisomers, esters, prodrugs, solvates and deuterated compounds of the compound according to the first aspect.

Specifically, the stereoisomer has a structure of:

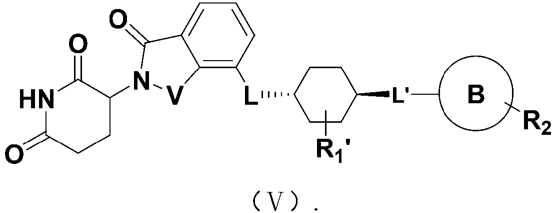

In some embodiments of the present disclosure, the stereoisomer has a structure of:

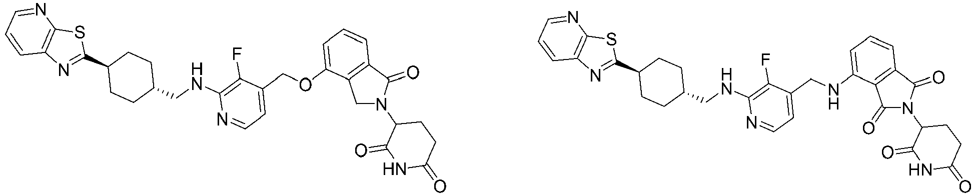

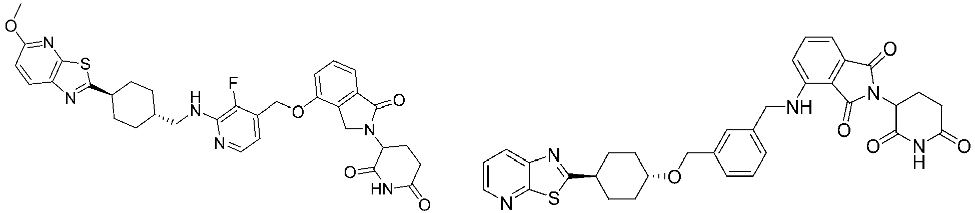

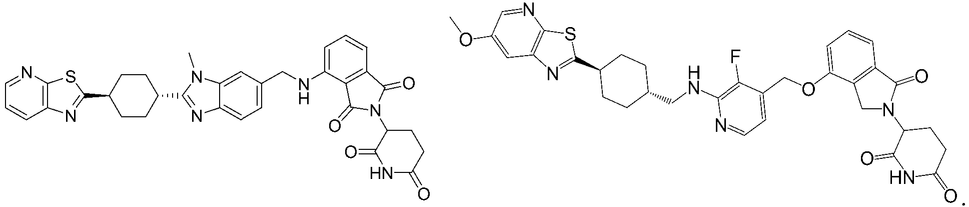

In a third aspect of the present disclosure, there is provided a pharmaceutical composition comprising the compound according to the first aspect, or a pharmaceutically acceptable salt, stereoisomer, ester, prodrug, solvate or deuterated compound thereof, and one or more pharmaceutically acceptable excipients.

Specifically, in a pharmaceutical composition, the compound of the first aspect, or a pharmaceutically acceptable salt, stereoisomer, ester, prodrug, solvate, and deuterated compound thereof, may be used alone or in combination with other types of active ingredients.

Specifically, the pharmaceutically acceptable excipient may be selected from one or more of: fillers, binders, lubricants, disintegrants, antioxidants, buffering agents, bacteriostatic agents, suspending agents, solubilizers, thickeners, stabilizers, preservatives, and the like.

Specifically, the pharmaceutical composition may be administered via any suitable route of administration, for example, gastrointestinal administration (e.g., oral, sublingual, or rectal administration) or parenteral administration (e.g., intravenous, intramuscular, intranasal, intraocular, intracerebral, intravaginal, intraperitoneal, transdermal, subcutaneous, intradermal, or respiratory tract administration).

Specifically, the pharmaceutical composition may be prepared into pharmaceutical formulations in the following forms: injections, syrups, elixirs, suspensions, powders, granules, tablets, capsules, lozenges, creams, ointments, lotions, gels, emulsions, and the like.

When preparing injections, any commonly used carrier in the art may be used, such as water, ethanol, propylene glycol, ethoxylated isostearyl alcohol, polyethoxylated isostearyl alcohol, and fatty acid esters of polyoxyethylene sorbitan. Furthermore, commonly used solubilizers and buffering agents may alternatively be added.

Specifically, the pharmaceutical composition is preferably in a unit dosage form. In this form, the formulation is subdivided into unit doses containing appropriate amounts of the active ingredient. The unit dosage form may be a capsule, a tablet, or any dosage form. In addition, the unit dosage form may be a packaged formulation, such as tablets, capsules, and powders packaged in vials or ampoules.

Specifically, the amount of the active ingredient in the unit dosage form formulation may vary or be adjusted from 0.1 mg to 1000 mg (e.g., 0.1, 1, 5, 10, 20, 40, 50, 100, 200, 400, 500, or 1000 mg), depending on the specific use and potency of the active ingredient. If desired, the composition may further contain other suitable therapeutic agents.

In a fourth aspect of the present disclosure, there is provided the use of the compound according to the first aspect, or a pharmaceutically acceptable salt, stereoisomer, ester, prodrug, solvate or deuterated compound thereof, in the preparation of a medicament for the prevention and/or treatment of IKZF1-related diseases.

Specifically, the disease is a disease for which the prevention and/or treatment is benefited by degradation of IKZF1, such as, but not limited to, a tumor, an autoimmune disease, an inflammatory disease, a disease related to pathogen infection, a neurodegenerative disease, and a xenoimmune disease.

Specifically, the tumor includes but is not limited to: adrenal cancer, anal cancer, angiosarcoma, appendiceal cancer, biliary tract cancer, bladder cancer, breast cancer, brain cancer, bronchial cancer, carcinoid tumor, cervical cancer, choriocarcinoma, chordoma, craniopharyngioma, colorectal cancer, connective tissue cancer, epithelial cancer, ependymoma, endothelioma, endometrial cancer, esophageal cancer, Ewing's sarcoma, eye cancer, gallbladder cancer, gastric cancer, gastrointestinal stromal tumor (GIST), germ cell cancer, head and neck cancer, hematological malignancy, hemangioblastoma, hypopharyngeal cancer, inflammatory myofibroblastic tumor, immunocellular amyloidosis, kidney cancer, liver cancer, lung cancer, leiomyosarcoma (LMS), muscle cancer, mesothelioma, myeloproliferative disease (MPD), neuroblastoma, neurofibroma, neuroendocrine cancer, osteosarcoma, ovarian cancer, papillary adenocarcinoma, pancreatic cancer, penile cancer, pinealoma, primitive neuroectodermal tumor (PNT), prostate cancer, rhabdomyosarcoma, salivary gland cancer, skin cancer, small intestine cancer, soft tissue sarcoma, sebaceous gland carcinoma, sweat gland cancer, synovioma, testicular cancer, thyroid cancer, urethral cancer, vaginal cancer, and vulvar cancer.

More specifically, the tumor is a hematological malignancy, such as leukemia, lymphoma, or multiple myeloma (MM).

Specifically, the leukemia may be chronic lymphocytic leukemia (CLL) (e.g., B-cell CLL or T-cell CLL), chronic myeloid leukemia (CML) (e.g., B-cell CML or T-cell CML), acute lymphoblastic leukemia (ALL) (e.g., B-cell ALL or T-cell ALL), acute myeloid leukemia (AML) (e.g., B-cell AML or T-cell AML), or acute monocytic leukemia, and in particular acute myeloid leukemia. Specifically, the leukemia may be relapsed, refractory, or drug-resistant.

Specifically, the lymphoma may be Hodgkin's lymphoma (HL) (e.g., B-cell HL or T-cell HL) and non-Hodgkin's lymphoma (NHL), e.g., B-cell NHL, such as diffuse large cell lymphoma (DLCL) (e.g., diffuse large B-cell lymphoma), follicular lymphoma, chronic lymphocytic leukemia/small lymphocytic lymphoma (CLL/SLL), mantle cell lymphoma (MCL), marginal zone B-cell lymphoma (e.g., mucosa-associated lymphoid tissue (MALT) lymphoma, nodal marginal zone B-cell lymphoma, or splenic marginal zone B-cell lymphoma), primary mediastinal B-cell lymphoma, Burkitt's lymphoma, lymphoplasmacytic lymphoma (i.e., Waldenström's macroglobulinemia), hairy cell leukemia (HCL), immunoblastic large cell lymphoma, precursor B-lymphoblastic lymphoma, and primary central nervous system (CNS) lymphoma, and T-cell NHL, such as precursor T-lymphoblastic lymphoma/leukemia, peripheral T-cell lymphoma (PTCL) (e.g., cutaneous T-cell lymphoma (CTCL) (such as mycosis fungoides and Sezary syndrome)), angioimmunoblastic T-cell lymphoma, extranodal natural killer T-cell lymphoma, enteropathy-type T-cell lymphoma, subcutaneous panniculitis-like T-cell lymphoma and anaplastic large cell lymphoma, and NK/T-cell lymphoma, and in particular diffuse large B-cell lymphoma (DLBCL).

In some embodiments of the present disclosure, the tumor is acute lymphoblastic leukemia (ALL).

Specifically, the autoimmune diseases include, but are not limited to: autoimmune diabetes (also known as Type I diabetes or insulin-dependent diabetes mellitus), systemic lupus erythematosus, systemic vasculitis, regional enteritis, cardiomyopathy, hemolytic anemia, fibromyalgia, Graves' disease, ulcerative colitis, Crohn's disease, multiple sclerosis, myasthenia gravis, myositis, neutropenia, psoriasis, chronic fatigue syndrome, juvenile arthritis, juvenile diabetes, scleroderma, psoriatic arthritis, Sjögren's syndrome, rheumatic fever, rheumatoid arthritis, sarcoidosis, idiopathic thrombocytopenic purpura (ITP), Hashimoto's disease, mixed connective tissue disease, interstitial cystitis, pernicious anemia, leukoencephalitis, alopecia areata, ankylosing spondylitis, primary biliary cirrhosis, anti-GBM nephritis, anti-TBM nephritis, antiphospholipid syndrome, polymyalgia rheumatica, polymyositis, autoimmune Addison's disease, chronic active hepatitis, vitiligo, psoriasis, autoimmune hyperlipidemia, autoimmune myocarditis, temporal arteritis, autoimmune thyroid disease, axonal and neuronal neuropathy, Behçet's disease, bullous pemphigoid, allergic asthma, osteoarthritis, Chagas' disease, uveitis, chronic inflammatory demyelinating polyneuropathy (CIDP), cicatricial pemphigoid/benign mucous membrane pemphigoid, Cogan's syndrome, congenital heart block, Coxsackie viral myocarditis, demyelinating neuropathy, dermatomyositis, discoid lupus, lens antigenic uveitis, polyarteritis nodosa, Dressler's syndrome, idiopathic mixed cryoglobulinemia, Evans syndrome, Goodpasture's syndrome, allergic rhinitis, infectious neuritis, hypogammaglobulinemia, inclusion body myositis, vesiculobullous dermatosis, Wegener's granulomatosis, Meniere's disease, Lambert-Eaton syndrome, Mooren's ulcer, atypical celiac disease, ocular cicatricial pemphigoid, pemphigus vulgaris, perivenous encephalomyelitis, post-pericardiotomy syndrome, scleritis, sperm/testis autoimmunity, stiff-person syndrome, subacute bacterial endocarditis (SBE), sympathetic ophthalmia, transverse myelitis and necrotizing myelopathy, Type I autoimmune polyglandular syndrome, Type II autoimmune polyglandular syndrome, pernicious anemia, and endometriosis, and the like.

In some embodiments of the present disclosure, the autoimmune disease is systemic lupus erythematosus (SLE).

In some embodiments of the present disclosure, the autoimmune disease is Type I diabetes or autoimmune thyroid disease.

Specifically, the inflammatory disease includes, but is not limited to: osteoarthritis, acute gout, multiple sclerosis, inflammatory bowel disease (such as Crohn's disease and ulcerative colitis), neuroinflammation, asthma, chronic obstructive airway disease, pneumonia, myositis, eczema, dermatitis, acne, cellulitis, occlusive disease, thrombosis, alopecia, nephritis, vasculitis, retinitis, uveitis, scleritis, sclerosing cholangitis, hypophysitis, thyroiditis, septic shock, systemic inflammatory response syndrome (SIRS), toxic shock syndrome, acute lung injury, acute respiratory distress syndrome (ARDS), acute renal failure, burns, pancreatitis (e.g., acute pancreatitis), post-surgical syndrome, sarcoidosis, Jarisch-Herxheimer reaction, encephalitis, myelitis, meningitis, malaria, and the like.

Specifically, the neurodegenerative disease includes, but is not limited to: Alzheimer's disease (AD), Parkinson's disease (PD), Huntington's disease (HD), amyotrophic lateral sclerosis (ALS), different types of spinocerebellar ataxia (SCA), Pick's disease, and the like.

Specifically, the pathogen may be a microorganism, a parasite (protozoa, helminths, etc.), or other vectors.

Specifically, the microorganism may be selected from one or more of: viruses, chlamydiae, rickettsiae, mycoplasmas, bacteria, spirochetes, fungi, and the like.

In one embodiment of the present disclosure, the pathogen is a virus, such as, but not limited to, Adenoviridae (e.g., adenovirus), Herpesviridae (e.g., Herpes Simplex Virus Type 1 (HSV1, oral herpes), HSV2 (genital herpes), Varicella-Zoster Virus (VZV, chickenpox), Epstein-Barr Virus (EBV), Cytomegalovirus (CMV)), Poxviridae (such as smallpox virus and vaccinia virus), Papovaviridae (e.g., Human Papillomavirus (HPV)), Parvoviridae (e.g., parvovirus B19), Hepadnaviridae (e.g., hepatitis B virus), Polyomaviridae (e.g., polyomavirus), Reoviridae (such as reovirus and rotavirus), Picornaviridae (such as enterovirus and foot-and-mouth disease virus), Caliciviridae (such as Norwalk virus and hepatitis E virus), Togaviridae (e.g., rubella virus), Arenaviridae (e.g., lymphocytic choriomeningitis virus), Retroviridae (such as Human Immunodeficiency Virus Type 1 (HIV-1), HIV-2, and Human T-Cell Leukemia Virus Type 1 (HTLV-1)), Flaviviridae (such as dengue virus, Zika virus, Japanese encephalitis virus, chikungunya virus, yellow fever virus, hepatitis C virus, and West Nile virus), Orthomyxoviridae (e.g., influenza virus (such as influenza A virus, influenza B virus, and influenza C virus)), Paramyxoviridae (such as Human Parainfluenza Virus (HPIV) Type 1, HPV type 2, HPV type 3, HPV type 4, Sendai virus, mumps virus, measles virus, respiratory syncytial virus, and Newcastle disease virus), Bunyaviridae (such as California encephalitis virus and hantavirus), Rhabdoviridae (e.g., rabies virus), Filoviridae (such as Ebola virus and Marburg virus), Coronaviridae (such as Human Coronavirus (HCoV)-229E, HCoV-OC43, HCoV-NL63, HCoV-HKU1, Severe Acute Respiratory Syndrome Coronavirus (SARS-CoV), Middle East Respiratory Syndrome Coronavirus (MERS-CoV), and SARS-CoV-2), Astroviridae (e.g., astrovirus), and Bornaviridae (e.g., Borna disease virus).

Specifically, the disease related to pathogen infection includes, but is not limited to: influenza, SARS, Coronavirus Disease 2019 (COVID-19), viral hepatitis (such as hepatitis A, hepatitis B, hepatitis C, and hepatitis D), Acquired Immunodeficiency Syndrome (AIDS), rabies, dengue fever, Ebola virus disease, and the like.

Specifically, the xenoimmune disease is rejection after organ transplantation.

In a fifth aspect of the present disclosure, there is provided a method for preventing and/or treating an IKZF1-related disease, including the step of administering to a subject in need thereof an effective amount of the compound of the first aspect, or a pharmaceutically acceptable salt, stereoisomer, ester, prodrug, solvate, and deuterated compound thereof, or the pharmaceutical composition of the third aspect.

Specifically, the disease is as described in the fourth aspect of the present disclosure.

Specifically, the subject is a mammal, and in particular a human.

### DETAILED DESCRIPTION

Unless otherwise defined, all scientific and technical terms used in the present disclosure have the same meaning as commonly understood by one of ordinary skill in the art to which the present disclosure pertains.

In the present disclosure, the term "aliphatic group" refers to a straight or branched hydrocarbon chain, or a cyclic hydrocarbon group (also referred to herein as "carbocycle", "alicyclic ring", or "cycloalkyl"), which is fully saturated or contains one or more units of unsaturation, and which is attached to the rest of the molecule by a single bond. Suitable aliphatic groups include, but are not limited to, straight or branched, substituted or unsubstituted alkyl, alkenyl, alkynyl, and combinations thereof, such as (cycloalkyl)alkyl, (cycloalkenyl)alkyl, (cycloalkyl)alkenyl, and the like. Typically, the aliphatic group contains from 1 to 10 (e.g., 1, 2, 3, 4, 5, 6, 7, 8, 9, 10) carbon atoms, preferably from 1 to 6 carbon atoms.

The term "alkyl" refers to a straight or branched hydrocarbon chain radical containing no unsaturated bonds, which is attached to the rest of the molecule by a single bond. Typically, the alkyl group contains from 1 to 10 (e.g., 1, 2, 3, 4, 5, 6, 7, 8, 9, 10) carbon atoms, preferably from 1 to 6 carbon atoms, such as methyl, ethyl, n-propyl, isopropyl, n-butyl, isobutyl, tert-butyl, n-pentyl, isopentyl, neopentyl, tert-pentyl, n-hexyl, isohexyl, and the like. In the present disclosure, C₀ alkyl refers to H; that is, C₀₋₁₀ alkyl (or C_{0-C10} alkyl) includes H and C₁₋₁₀ alkyl (or C₁-C₁₀ alkyl).

The term "alkylene" refers to a hydrocarbyl (divalent alkyl) derived from an alkane molecule by the removal of two hydrogen atoms. It may be linear or branched and is attached to the rest of the molecule by a single bond. The typical alkylene herein has 1 to 10 (e.g., 1, 2, 3, 4, 5, 6, 7, 8, 9, or 10) carbon atoms, preferably 1 to 6 carbon atoms, such as methylene (-CH₂-), ethylene, propylene, and butylene. In the present disclosure, C₀ alkylene refers to a single bond. In other words, C₀₋₁₀ alkylene (or C₀-C₁₀ alkylene) includes a single bond and C₁₋₁₀ alkylene (or C₁-C₁₀ alkylene).

The term "cycloalkyl" refers to an alicyclic hydrocarbon, e.g., containing 1 to 4 monocyclic and/or fused rings, or containing 3-18 carbon atoms, preferably 3-10 (e.g., 3, 4, 5, 6, 7, 8, 9, or 10) carbon atoms, such as cyclopropyl, cyclobutyl, cyclopentyl, cyclohexyl, and adamantyl.

The term "alkoxy" refers to a substituent formed by replacing the hydrogen in a hydroxyl group with an alkyl group, such as an alkoxy group containing 1-10 carbon atoms, for example, methoxy, ethoxy, propoxy, butoxy, and the like.

The term "alkylamino" refers to a substituent formed by replacing one or two hydrogens in an amino group (-NH2) with an alkyl group(s), such as an alkylamino group containing 1-10 carbon atoms, for example

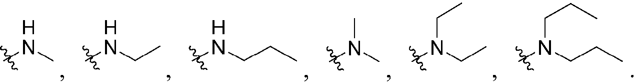

The term "halogen" refers to fluorine, chlorine, bromine, or iodine.

The term "haloalkyl" refers to a group formed by replacing one or more hydrogens in an alkyl group with halogen atoms (e.g., fluorine, chlorine, bromine, or iodine), such as -CHF2, - CH2F, -CF3, -CH2-CF3, -CH2CH2-CF3, and -CH2CH2CH2-CF3.

The term "aryl" refers to a monocyclic or polycyclic radical, including polycyclic radicals containing monocyclic aryl groups and/or fused aryl groups, such as containing 1-3 monocyclic or fused rings and 6-18 (e.g., 6, 8, 10, 12, 14, 16, 18) carbocyclic atoms. The C₆-C₁₂ aryl described in the present disclosure refers to an aryl group containing 6-12 carbocyclic atoms, such as phenyl, naphthyl, biphenyl, indenyl, and the like.

The term "heterocyclyl" refers to a 3- to 18-membered non-aromatic ring group containing 2 to 17 carbon atoms and 1 to 10 heteroatoms. The heterocyclyl may be a monocyclic, bicyclic, tricyclic, or tetracyclic ring system, which may comprise fused, spiro, or bridged ring systems. The heterocyclyl may be partially saturated (heteroaryl) or fully saturated (heterocycloalkyl). Suitable heteroaryl groups in the compounds of the present disclosure contain 1, 2, or 3 kinds of heteroatoms selected from N, O, S, and P atoms. The heteroaryl groups include, for example, coumarin, including 8-coumarinyl, quinolinyl, including 8-quinolinyl, isoquinolinyl, pyridyl, pyrazinyl, pyrazolyl, pyrimidinyl, furyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuryl, benzofurazanyl, benzothienyl, benzothiazolyl, benzoxazolyl, quinazolinyl, quinoxalinyl, naphthyridinyl, and furopyridinyl. Suitable heterocycloalkyl groups in the compounds of the present disclosure contain 1, 2, or 3 kinds of heteroatoms selected from N, O, or S atoms. The heterocycloalkyl groups include, for example, pyrrolidinyl, tetrahydrofuranyl, dihydrofuryl, tetrahydrothienyl, tetrahydrothiopyranyl, piperidinyl, morpholinyl, thiomorpholinyl, oxathianyl, piperazinyl, azetidinyl, oxetanyl, thietanyl, homopiperidinyl, oxiranyl, thiiranyl, azepinyl, oxazepanyl, diazepinyl, triazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, pyrazolidinyl, imidazolinyl, imidazolidinyl, 3-azabicyclo[3.1.0]hexyl, 3-azabicyclo[4.1.0]heptyl, 3H-indolyl, and quinolizinyl. In the present disclosure, for an optionally substituted heterocyclyl, the position of substitution may be any suitable carbon atom or heteroatom. For example, for 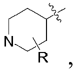 the substitution position of R may be any suitable carbon atom or nitrogen atom, which may be, for example,

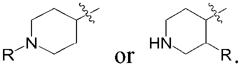

In the present disclosure, "D" refers to deuterium; "substituted with deuterium" means replacing one or more hydrogen atoms with a corresponding number of deuterium atoms.

It should be recognized that there are some variations in the abundance of natural isotopes in the synthesized compounds depending on the source of the chemical materials used in the synthesis. Therefore, the compounds of the present disclosure will inherently contain a small amount of deuterated isotopologues. Despite this variation, the concentration of stable hydrogen and carbon isotopes in this natural abundance is still very low and insignificant compared to the degree of stable isotopic substitution of the compounds of the present disclosure, see, for example, Wada, E et al., Seikagaku, 1994, 66: 15; Gannes, LZ et al., Comp Biochem Physiol Mol Integr Physiol, 1998, 119: 725.

In the compounds of the present disclosure, any atom not specified as deuterium is present in its natural isotopic abundance. Unless otherwise stated, when a position is specifically designated as "H" or "hydrogen", the position should be understood to have hydrogen in its natural abundance isotopic composition. Similarly, unless otherwise stated, when a position is specifically designated as "D" or "Deuterium", the position should be understood to have deuterium with an abundance at least 3000-fold higher than the natural abundance of deuterium (which is 0.015%) (i.e., at least 45% deuterium incorporation).

The term "isotopic enrichment ratio" herein refers to the ratio between the isotopic abundance of a particular isotope and its natural abundance.

In other embodiments, the compound of the present disclosure has an isotopic enrichment ratio for each specified deuterium atom of at least 3500 (52.5% deuterium incorporation at each specified deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The term " isotopologue" refers to a substance in which the chemical structure differs from the specific compound of the present disclosure only in its isotopic composition.

The term "pharmaceutically acceptable salts" includes acid addition salts and base addition salts.

The term "acid addition salt" includes, but is not limited to, salts from inorganic acids (such as hydrochloric acid, nitric acid, phosphoric acid, sulfuric acid, hydrobromic acid, hydroiodic acid, and phosphonic acid), and salts from organic acids (such as aliphatic monocarboxylic acids and dicarboxylic acids, phenyl-substituted alkanoic acids, hydroxyalkanoic acids, alkanedioic acids, aromatic acids, and aliphatic and aromatic sulfonic acids). Therefore, these salts include but are not limited to sulfate, pyrosulfate, hydrogen sulfate, sulfite, bisulfite, nitrate, phosphate, monohydrogen phosphate, dihydrogen phosphate, metaphosphate, pyrophosphate, hydrochloride, hydrobromide, iodate, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, tartrate, and methanesulfonate, and also contains amino acid salts such as arginine, gluconate, and galacturonate. The acid addition salt can be prepared by contacting the free base form with a sufficient amount of the desired acid in a conventional manner to form a salt. The free base form can be regenerated by contacting the salt form with the base, and the free base is separated in a conventional manner.

The term "alkali addition salt" refers to a salt formed with a metal or amine, such as hydroxides of alkali metals and alkaline earth metals, or formed with an organic amine. Examples of metals used as cations include, but are not limited to, sodium, potassium, magnesium, and calcium. Examples of suitable amines include, but are not limited to, N,N ' - dibenzylethylenediamine, chloroprocaine, choline, diethanolamine, ethylenediamine (ethane-1,2-diamine), N-methylglucosamine, and procaine. The alkali addition salt can be prepared by contacting the free acid form with a sufficient amount of the desired base in a conventional manner to form a salt. The free acid form can be regenerated by contacting the salt form with the acid, and the free acid is separated in a conventional manner.

The term "stereoisomer" includes the existence of enantiomers, diastereomers, and geometric isomers. Some compounds of the present disclosure have cyclic hydrocarbyl that can be substituted on more than one carbon atom, in which case all their geometric forms, including cis and trans, and mixtures thereof, are within the scope of the present disclosure.

The term "solvate" refers to the physical combination of the compound of the present disclosure with one or more solvent molecules. The physical bonding includes various degrees of ionic and covalent bonding, including hydrogen bonding. In some cases, solvates can be separated, for example, when one or more solvent molecules are incorporated into the lattice of a crystalline solid. The solvate includes a solution phase and a separable solvate. Representative solvates include ethanolates and methanolates.

The term "prodrug" refers to a form of the compound of formula I that is suitable for administration to a patient without excessive toxicity, irritation, and allergic reactions and is effective for its application purpose, including acetal, ester, and zwitterionic forms. The prodrug is transformed in the body, such as by hydrolysis in the blood, to obtain the parent compound.

The terms "patient" or "subject" and the like are used interchangeably herein and refer to any animal or its cells treated according to the methods described herein, whether in vitro or in situ. Specifically, the animals include mammals, such as rats, mice, guinea pigs, rabbits, dogs, monkeys, or humans, especially humans.

The term "treatment" refers to the prevention, cure, reversal, alleviation, reduction, minimization, suppression, cessation, and/or stopping of one or more clinical symptoms of a disease after its onset.

The term "prevention" refers to treatment administered prior to the onset of a disease in order to avoid, minimize, or make the occurrence or progression of the disease more difficult.

The term "tumor" refers to an abnormal mass of tissue, the growth of which exceeds and is uncoordinated with that of normal tissues. Tumors can be "benign" or "malignant", depending on the following characteristics: degree of cell differentiation (including morphology and function), growth rate, local invasion and metastasis. "Benign tumors" are usually well-differentiated and are characterized by slower growth compared to malignant tumors, remaining confined to their site of origin. In addition, benign tumors do not have the ability to infiltrate, invade, or metastasize to distant sites. In some cases, certain "benign" tumors may later develop into malignant tumors, which may be caused by additional genetic alterations in subpopulations of the neoplastic cells. Such tumors are referred to as "precancerous tumors." "Malignant tumors" are usually poorly differentiated (anaplastic) and have characteristic rapid growth, accompanied by progressive infiltration, invasion, and destruction of surrounding tissues. In addition, malignant tumors often have the ability to metastasize to distant sites.

The term "cancer" refers to a malignant tumor (Stedman's Medical Dictionary, 25th ed.; Hensyl ed.; Williams & Wilkins: Philadelphia, 1990).

The term "autoimmune disease" refers to a disease caused by the body's immune response to its own antigens, resulting in damage to its own tissues.

The term "inflammation" is the body's defensive response to stimuli, manifested as redness, swelling, heat, pain, and dysfunction; it can be infectious inflammation caused by infection or non-infectious inflammation not caused by infection, such as inflammation caused by immune responses (such as various types of hypersensitivity reactions and inflammation associated with autoimmune diseases). The term "inflammatory disease" refers to a disease with inflammation.

The term "disease related to pathogen infection" mainly refers to a disease caused by pathogen infection, which includes symptoms of bodily injury and infection response manifestations caused by pathogen invasion. The pathogen may be a microorganism (such as viruses, chlamydiae, rickettsiae, mycoplasmas, bacteria, spirochetes, and fungi), a parasite (such as protozoa and helminths), or other vectors. In particular, the pathogen is a virus, such as, but not limited to, Adenoviridae (e.g., adenovirus), Herpesviridae (e.g., HSV1 (oral herpes), HSV2 (genital herpes), VZV (chickenpox), EBV, CMV), Poxviridae (such as smallpox virus and vaccinia virus), Papovaviridae (e.g., HPV), Parvoviridae (e.g., parvovirus B19), Hepadnaviridae (e.g., hepatitis B virus), Polyomaviridae (e.g., polyomavirus), Reoviridae (such as reovirus and rotavirus), Picornaviridae (such as enterovirus and foot-and-mouth disease virus), Caliciviridae (such as Norwalk virus and hepatitis E virus), Togaviridae (e.g., rubella virus), Arenaviridae (e.g., lymphocytic choriomeningitis virus), Retroviridae (such as HIV-1, HIV-2, and HTLV-1), Flaviviridae (such as dengue virus, Zika virus, Japanese encephalitis virus, chikungunya virus, yellow fever virus, hepatitis C virus, and West Nile virus), Orthomyxoviridae (e.g., influenza virus (such as influenza A virus, influenza B virus, and influenza C virus)), Paramyxoviridae (such as HPIV type 1, HPV type 2, HPV type 3, HPV type 4, Sendai virus, mumps virus, measles virus, respiratory syncytial virus, and Newcastle disease virus), Bunyaviridae (such as California encephalitis virus and hantavirus), Rhabdoviridae (e.g., rabies virus), Filoviridae (such as Ebola virus and Marburg virus), Coronaviridae (such as HCoV-229E, HCoV-OC43, HCoV-NL63, HCoV-HKU1, SARS-CoV, MERS-CoV, and SARS-CoV-2), Astroviridae (e.g., astrovirus), and Bornaviridae (e.g., Borna disease virus).

The disclosures of various publications, patents and published patent specifications cited herein are incorporated herein by reference in their entirety.

The technical solution of the present disclosure will be clearly and completely described below with reference to the embodiments. Obviously, the embodiments described are some rather than all of the embodiments of the present disclosure. Based on the embodiments described herein, all other embodiments obtained by those of ordinary skill in the art without creative work are within the scope of protection of the present disclosure.

### Compound Preparation Examples:

### Example 1: Preparation of Compound 1

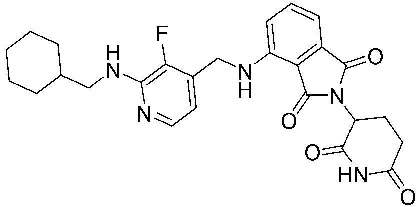

The synthesis steps were as follows:

### Step 1:

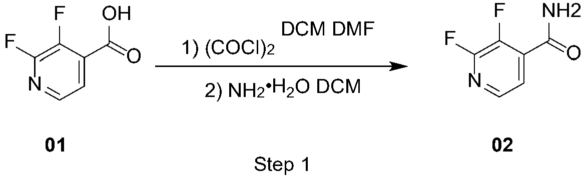

At room temperature, compound 01 (5.25 g, 33 mmol) was dissolved in dichloromethane (50 mL). At 0°C, N,N-dimethylformamide (DMF) (1 drop) was added, followed by (COCl)₂ (16.8 g, 132 mmol). The mixture was reacted with stirring at room temperature for 10 minutes. After 10 minutes, the reaction mixture was concentrated to obtain a crude product. The crude product was dissolved in dichloromethane (40 mL), and aqueous ammonia (10 mL) was added. The mixture was reacted with stirring at room temperature for 2 hours. The reaction mixture was concentrated to obtain a crude product. The crude product was purified by column chromatography (petroleum ether:ethyl acetate = 1:20) to obtain a yellow solid **02** (4.85 g, yield: 93.09%). LC-MS: 156.8 [M-H]⁺

### Step 2:

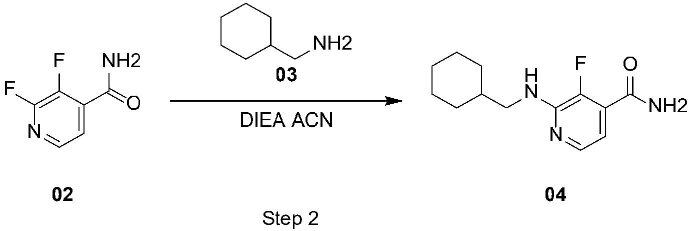

At room temperature, the yellow solid **02** (1 g, 6.3 mmol) was dissolved in acetonitrile (15 mL), and N,N-Diisopropylethylamine(DIEA) (2.45 g, 19.0 mmol) and compound **03** (1.43 g, 12.7 mmol) were added. The mixture was reacted at 70°C for 16 hours. After the reaction was complete, the reaction mixture was concentrated to obtain a crude product. The crude product was diluted with 30 mL of water, extracted with ethyl acetate (30 mL × 3), and washed with a saturated salt solution (20 mL × 3). The organic phases were mixed, dried over anhydrous sodium sulfate, and then concentrated to obtain a crude product. The crude product was purified by column chromatography (petroleum ether:ethyl acetate = 5:1) to obtain a pale yellow solid 04 (600 mg, yield: 37.74%). LC-MS: 252.2.0 [M+H]⁺

### Step 3:

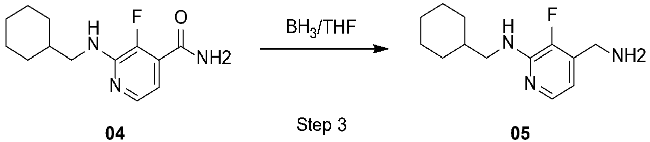

At room temperature, BH₃/tetrahydrofuran (THF) (20 mL) was heated to 40°C, and the pale yellow solid **04** (560 mg, 2.23 mmol) was added. The mixture was reacted with stirring at 40°C for 4 hours. After being cooled to room temperature, the reaction mixture was added with methanol for quenching. The reaction mixture after quenching was concentrated to obtain a crude product. The crude product was purified by column chromatography (dichloromethane:methanol = 20:1) to obtain a white solid **05** (170 mg, yield: 32.15%). LC-MS: 238.1 [M+H]⁺

### Step 4:

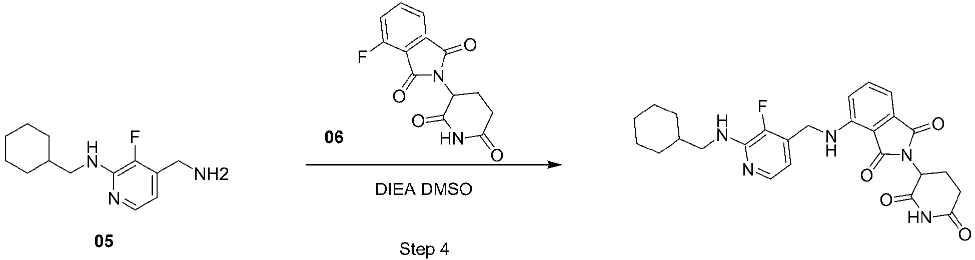

At room temperature, the white solid 05 (150 mg, 0.6 mmol) and compound 06 (350 mg, 1.3 mmol) were dissolved in dimethylsulfoxide (DMSO) (2 mL), and DIEA (345 mg, 1.9 mmol) was added. The mixture was reacted under microwave irradiation at 100°C for 2 hours. After the reaction was complete, the crude product was purified by reversed-phase column chromatography to obtain the title compound, a yellow-green solid (18.5 mg, yield: 6%). LC-MS: 494.1 [M+H]⁺

¹H NMR (400 MHz, DMSO-*d*₆) δ 11.09 (s, 1H), 7.69 (d, J= 5.1 Hz, 1H), 7.54 (t, J= 8.0 Hz, 1H), 7.17 - 7.10 (m, 1H), 7.06 (d, *J =* 7.2 Hz, 1H), 6.96 (d, *J* = 8.5 Hz, 1H), 6.65 - 6.55 (m, 1H), 6.40 (t, *J=* 4.8 Hz, 1H), 5.07 (dd, *J=* 12.9, 5.4 Hz, 1H), 4.57 (d, *J=* 6.6 Hz, 2H), 3.16 (t, *J =* 6.3 Hz, 2H), 2.95 - 2.82 (m, 1H), 2.70 - 2.55 (m, 2H), 2.10 - 2.00 (m, 1H), 1.75 - 1.55 (m, 6H), 1.20 - 1.08 (m, 3H), 0.95 - 0.82 (m, 2H).

### Example 2: Preparation of Compound 2

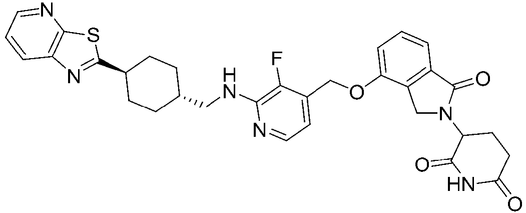

The synthesis steps were as follows:

### Step 1:

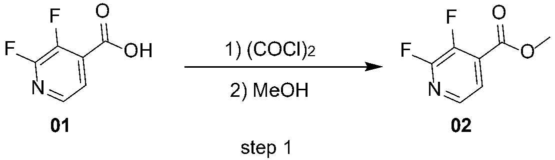

At room temperature, compound **01** (20 g, 125.8 mmol) was dissolved in dichloromethane (200 mL). At 0°C, DMF (5 d) was added, followed by (COCl)₂ (31.9 g, 251.5 mmol). The mixture was reacted with stirring at room temperature for 1 hour. After 1 hour, the reaction mixture was concentrated to obtain a crude product. The crude product was dissolved in methanol (200 mL). The mixture was reacted with stirring at room temperature for 16 hours. The reaction mixture was concentrated to obtain a crude product. The crude product was purified by column chromatography (petroleum ether:ethyl acetate = 10:1) to obtain a white solid 02 (18 g, yield: 82.9%). TLC (PE:EA=5:1)

### Step 2:

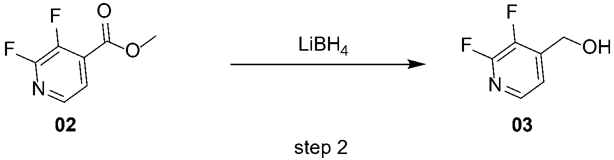

At room temperature, the white solid **02** (18 g, 104 mmol) was dissolved in methanol (200 mL). At 0°C, LiBH₄ (3.4 g, 156 mmol) was added. The mixture was reacted at room temperature for 1 hour. After the reaction was complete, the reaction mixture was added with water for quenching, extracted with dichloromethane (150 mL × 3), and washed with a saturated salt solution (100 mL × 2). The organic phases were mixed, dried over anhydrous sodium sulfate, and then concentrated to obtain a crude product. The crude product was purified by column chromatography (petroleum ether:ethyl acetate = 5:1) to obtain a white solid **03** (11 g, yield: 73.33%). LC-MS: 313.0 [2M+Na]⁺

### Step 3:

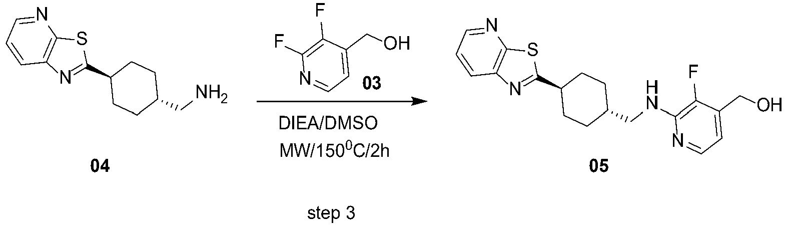

At room temperature, compound **04** (8 g, 32.4 mmol) and the white solid 03 (5.6 g, 38.9 mmol) were dissolved in DMSO (40 mL), and DIEA (20.9 g, 162 mmol) was added. The mixture was reacted with stirring under microwave irradiation at 150°C for 2 hours. After being cooled to room temperature, the reaction mixture was diluted with water (400 mL), extracted with ethyl acetate (400 mL × 3), and washed with a saturated salt solution (100 mL × 3). The organic phases were mixed, dried over anhydrous sodium sulfate, and then concentrated to obtain a crude product. The crude product was purified by column chromatography (petroleum ether:ethyl acetate = 2:3) to obtain a white solid **05** (3.6 g, yield: 30.0%). LC-MS: 373.0 [M+H]⁺

### Step 4:

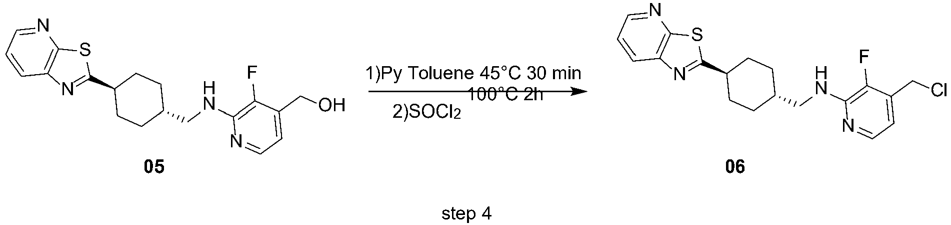

At room temperature, the white solid **05** (3.6 g, 9.68 mmol) was dissolved in dichloromethane (10 mL) and methylbenzene (20 mL), and pyridine (306 mg, 3.9 mmol) was added. The mixture was reacted at room temperature for 30 minutes. After 30 minutes, SOCl₂ (2.3 g, 19.4 mmol) was added dropwise slowly. The mixture was reacted with stirring at 100°C for 2 hours. After the reaction was complete, the reaction mixture was concentrated to obtain a crude product which was purified by column chromatography (dichloromethane:methanol = 10:1) to obtain a yellow solid **06** (2.4 g, yield: 63.66%). LC-MS: 391.1 [M+H]⁺

### Step 5:

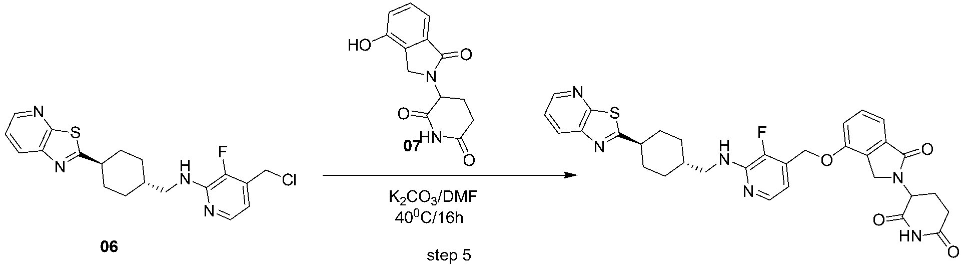

At room temperature, the yellow solid **06** (2.4 g, 6.15 mmol) and compound **07** (2.4 g, 9.23 mmol) were dissolved in DMF (10 mL), and K₂CO₃ (5.09 g, 36.92 mmol) was added. The mixture was reacted at 40°C for 16 hours. After the reaction was complete, the crude product was purified by reversed-phase column chromatography to obtain the title compound, a white solid (640.36 mg, yield: 17.3%). LC-MS: 615.0 [M+H]⁺

¹H NMR (400 MHz, DMSO-d6) δ 10.96 (s, 1H), 8.56 (dd, *J =* 4.6, 1.5 Hz, 1H), 8.32 (dd, *J* = 8.2, 1.5 Hz, 1H), 7.82 (d, *J =* 5.1 Hz, 1H), 7.56 - 7.49 (m, 2H), 7.35 (t, *J =* 7.3 Hz, 2H), 6.80 (s, 1H), 6.64 (t, *J =* 4.8 Hz, 1H), 5.28 (s, 2H), 5.11 (dd, *J* = 13.3, 5.0 Hz, 1H), 4.35 (dd, *J* = 65.2, 17.5 Hz, 2H), 3.15 - 3.8 (m, 2H), 2.94 - 2.88 (m, 1H), 2.68 - 2.66 (m, 1H), 2.60 - 2.54 (m, 1H), 2.46 - 2.40 (m, 1H), 2.33 - 2.30 (m, 1H), 2.24 - 2.18 (m, 2H), 2.02 - 1.96 (m, 1H), 1.95 - 1.90 (m, 2H), 1.61 - 1.52 (m, 2H), 1.21 - 1.13 (m, 2H).

### Example 3: Preparation of Compound 3

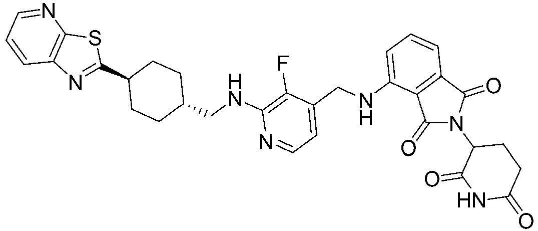

The synthesis steps were as follows:

### Step 1:

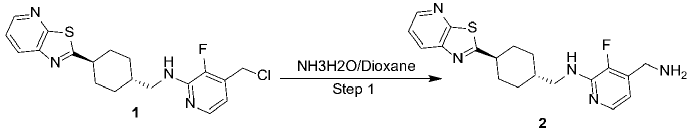

At room temperature, compound **1** (4.0 g, 10.23 mmol) (prepared in Example 2) was dissolved in dioxane (10 mL), and then aqueous ammonia (10 mL) was added. The mixture was reacted under sealed conditions for 16 hours. The residue obtained by spin-drying of the reaction mixture was purified by flash silica gel column chromatography (methanol/dichloromethane = 0-20%) to obtain a yellow solid as the target product (2.0 g, yield: 52.61%). LC-MS: 371.2 [M+H]⁺.

### Step 2:

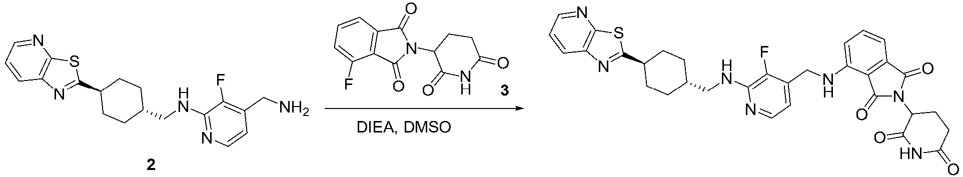

At room temperature, compound **2** (2.0 g, 5.38 mmol), compound **3** (2.23 g, 8.08 mmol), and N,N-diisopropylethylamine (2.8 g, 21.52 mmol) were dissolved in anhydrous dimethylsulfoxide (10 mL). The reaction mixture was reacted under microwave irradiation at 120°C for 2 hours. The mixture was separated by reverse-phase preparative liquid chromatography (C18, 5-100% acetonitrile/water as a mobile phase, and 0.1% formic acid). The resulting product was lyophilized by adding dilute hydrochloric acid to obtain the title compound (450 mg, yield: 13.3%, a yellow solid). LC-MS: 628.2 [M+H]⁺

¹H NMR (400 MHz, DMSO-d6) δ 11.11 (s, 1H), 8.57 (d, *J* = 4.4 Hz, 1H), 8.32 (d,*J* = 8.1 Hz, 1H), 7.71 (d, *J* = 5.9 Hz, 1H), 7.56 (dd, *J* = 11.4, 5.6 Hz, 2H), 7.36 - 7.20 (m, 1H), 7.09 (d, *J* = 7.1 Hz, 1H), 6.98 (d, *J* = 8.6 Hz, 1H), 6.61 (dd, *J* = 7.6, 5.5 Hz, 1H), 5.09 (dd, *J* = 12.7, 5.3 Hz, 1H), 4.69 - 4.64 (m, 2H), 3.36 - 3.26 (m, 3H), 3.19 - 3.08 (m, 1H), 2.98 - 2.84 (m, 1H), 2.62 - 2.54 (m, 2H), 2.22 - 2.19 (m, 2H), 2.09 - 2.02 (m, 1H), 2.00 -1.90 (m, 2H), 1.83 - 1.66 (m, 1H), 1.67 - 1.50 (m, 2H), 1.34 - 1.12 (m, 2H).

### Example 4: Preparation of Compound 4

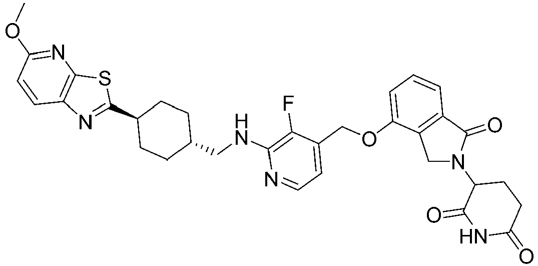

The synthesis steps were as follows:

### Step 1:

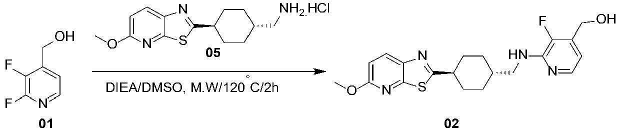

At room temperature, compound **01** (1.25 g, 8.62 mmol), compound **05** (2 g, 7.22 mmol), and N-ethyldiisopropylamine (12.6 mL, 72.47 mmol) were mixed in dimethylsulfoxide (20 mL). The reaction mixture was reacted under microwave irradiation at 120°C for 2 hours. The reaction mixture was added with 160 mL of water, and extracted with 50 ml of dichloromethane for three times. The organic phase was washed with 30 mL of water twice and 30 mL of salt solution once, then dried over anhydrous sodium sulfate, filtered, spin-dried, and purified by column chromatography to obtain a yellow oil **02** (313 mg, yield: 9%). LC-MS: 403.0 [M+H]⁺.

### Step 2:

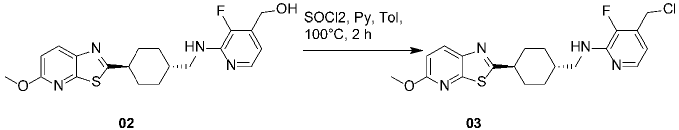

At room temperature, the yellow oil **02** (434 mg, 1.08 mmol), thionyl chloride (0.16 mL, 2.2 mmol), and pyridine (35 µL, 0.43 mmol) were mixed in anhydrous toluene (5 mL). The reaction mixture was reacted with stirring at 100°C for 2 hours. The reaction mixture was spin-dried and then purified by column chromatography to obtain a yellow solid 03 (289 mg, crude product). Then, 100 mg of the yellow crude product was purified by thin-layer chromatography to obtain a yellow oil **03** (70 mg). LC-MS: 421.0 [M+H]⁺, TLC: petroleum ether:ethyl acetate = 5:1, Rf = 0.3.

### Step 3:

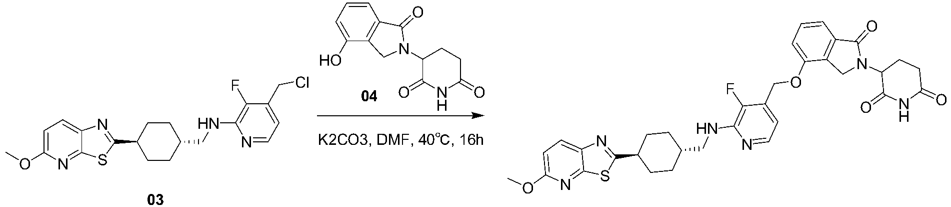

At room temperature, the yellow oil **03** (70 mg, 0.167 mmol), compound **04** (65 mg, 0.25 mmol), and potassium carbonate (138 mg, 0.998 mmol) were mixed in anhydrous *N,N-*dimethylformamide (0.5 mL). The reaction mixture was reacted with stirring at 40°C for 16 hours. By reversed-phase preparative liquid chromatography, the title compound was obtained as a white solid (2.04 mg, yield: 2%). LC-MS: 645.3 [M+H]⁺.

¹H NMR (400 MHz, DMSO-d6) δ 10.97 (s, 1H), 8.46 (s, 1H), 8.23 (d, *J =* 8.8 Hz, 1H), 7.78 (d, *J =* 5.1 Hz, 1H), 7.51 (t, *J =* 7.8 Hz, 1H), 7.35 (dd, *J =* 11.6, 7.8 Hz, 2H), 6.96 (d, *J =* 8.8 Hz, 1H), 6.82 - 6.75 (m, 1H), 6.63 (t, *J* = 4.9 Hz, 1H), 5.27 (s, 2H), 5.10 (dd, *J =* 13.2, 4.9 Hz, 1H), 4.35 (dd, *J =* 64.4, 17.6 Hz, 2H), 3.93 (s, 3H), 3.60 - 3.50 (m, 2H), 3.31 - 3.28 (m, 2H), 3.04 - 2.87 (m, 2H), 2.64 - 2.57 (m, 1H), 2.06 - 1.85 (m, 4H), 1.69 - 1.61 (m, 2H), 1.53 - 1.40 (m, 2H).

### Example 5: Preparation of Compound 5

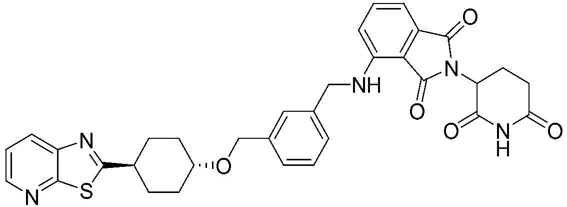

The synthesis steps were as follows:

### Step 1:

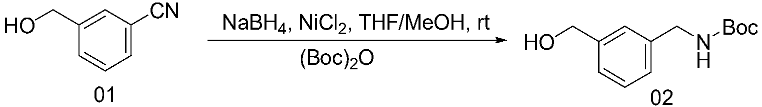

Under an ice bath, sodium borohydride (1.99 g, 52.5 mmol) was added to a solution of compound 01 (1 g, 7.51 mmol), anhydrous nickel chloride (973 mg, 7.51 mmol), and di-tert-butyl dicarbonate (3.278 g, 15.02 mmol) in MeOH (48 mL) and THF (32 mL). The mixture was reacted at room temperature for 12 hours. The reaction mixture was added with water for quenching, and extracted with ethyl acetate (50 mL × 3). The organic phases were mixed, washed with a saturated salt solution (50 mL × 2), dried over anhydrous sodium sulfate, spin-dried, and purified by column chromatography (ethyl acetate:petroleum ether = 1:2) to obtain a colorless oily liquid (1.12 g, yield: 62.6%). LC-MS: 260.3 [M+H]⁺

### Step 2:

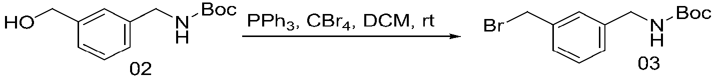

At room temperature, compound **02** (300 mg, 1.264 mmol) and CBr₄ (503 mg, 1.517 mmol) were added to a solution of dichloromethane (DCM) (5 mL). Under argon protection, triphenylphosphine (398 mg, 1.517 mmol) was added under the ice bath. The mixture was reacted at room temperature for 12 hours. The reaction mixture was added with water for quenching, and extracted with ethyl acetate (50 mL × 3). The organic phases were mixed, washed with a saturated salt solution (50 mL × 2), dried over anhydrous sodium sulfate, spin-dried, and purified by column chromatography (ethyl acetate:petroleum ether = 1:2) to obtain a white solid (277 mg, yield: 73.3 %). LC-MS: 244.1 [M+H]⁺

### Step 3:

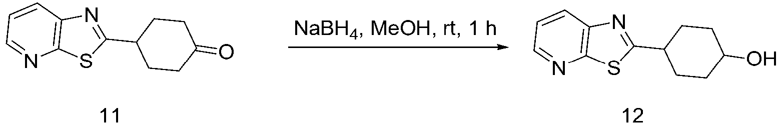

At room temperature, sodium borohydride (146.52 mg, 3.873 mmol) was added to a solution of compound 11 (300 mg, 1.291 mmol) in MeOH (10 mL). The mixture was reacted at room temperature for 1 hour. The reaction mixture was added with saturated ammonium chloride under the ice bath for quenching, and extracted with ethyl acetate (10 mL × 3). The organic phases were mixed, washed with a saturated salt solution (10 mL × 2), dried over anhydrous sodium sulfate, spin-dried, and purified by column chromatography (methanol:dichloromethane = 1:10) to obtain a white oily liquid (172 mg, yield: 56.9%). LC-MS: 235.2 [M+H]⁺

### Step 4:

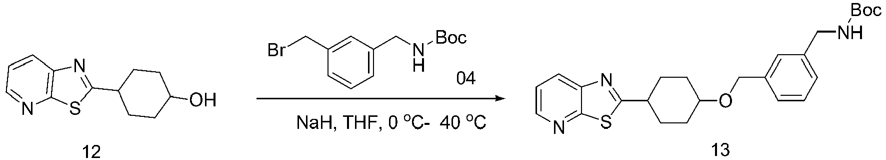

Under the ice bath, sodium hydride (42.624 mg, 1.776 mmol) was added to a solution of an intermediate 12 (104 mg, 0.444 mmol) and an intermediate 04 (200 mg, 0.666 mmol) in THF (10 mL). The mixture was reacted at 40°C for 2 hours. The reaction mixture was added with a saturated aqueous ammonium chloride solution for quenching, and extracted with ethyl acetate (10 mL × 3). The organic phases were mixed, washed with a saturated salt solution (10 mL × 2), dried over anhydrous sodium sulfate, spin-dried, and purified by column chromatography (ethyl acetate:petroleum ether = 1:1) to obtain a yellow solid (133 mg, yield: 66.1 %). LC-MS: 454.3 [M+H]⁺

### Step 5:

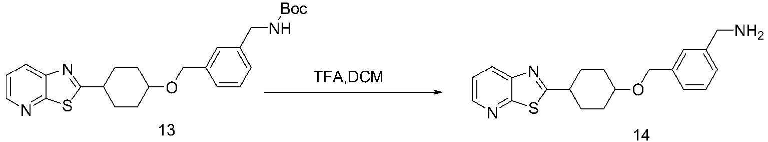

At room temperature, trifluoroacetic acid (2 mL) was added to a solution of compound 13 (90 mg, 0.199 mmol) in dichloromethane (2 mL). The mixture was reacted at room temperature for 12 hours, then spin-dried, and purified by reverse-phase chromatography (acetonitrile/water = 35%, 0.3% FA) to obtain a purple solid (20 mg, yield: 28.5%). LC-MS: 354.3 [M+H]⁺

### Step 6:

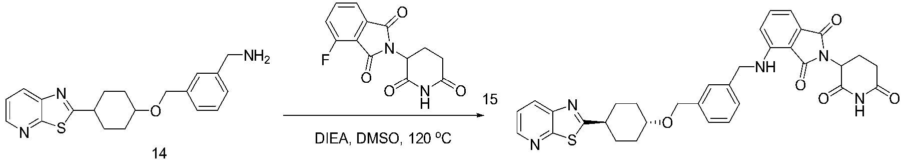

Compound 14 (20 mg, 0.0566 mmol), compound 15 (15.634 mg, 0.0566 mmol), N,N-diisopropylethylamine (21.94 mg, 0.1698 mmol), and dimethylsulfoxide (1 mL) were added to a 100 mL single-neck flask. The mixture was reacted at 120°C for 12 hours. The reaction mixture was purified by preparative high-performance liquid chromatography to obtain the title compound, a yellow solid (0.91 mg, yield: 2.6%). LC-MS: 610.3 [M+H]⁺

¹H NMR (400 MHz, DMSO-*d*₆) δ 11.11 (s, 1H), 7.73 (s, 1H), 7.53 - 7.46 (m, 1H), 7.38 (s, 1H), 7.27 - 7.22 (m, 3H), 7.12 (s, 1H), 7.04 - 6.98 (m, 2H), 6.91 (t, *J =* 5.9 Hz, 2H), 5.11 - 5.06 (m, 1H), 4.52 (d, *J* = 5.9 Hz, 2H), 4.38 (s, 2H), 2.88 (d, *J* = 11.6 Hz, 2H), 2.59 (d, *J* = 22.9 Hz, 4H), 2.01 (dd, *J* = 14.8, 7.1 Hz, 4H), 1.82 - 1.72 (m, 2H), 1.46 (s, 2H).

### Example 6: Preparation of Compound 6

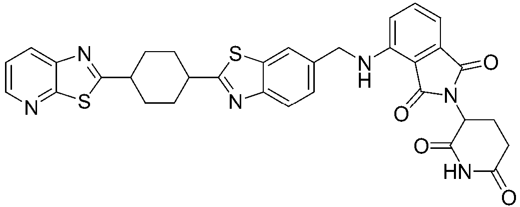

The synthesis steps were as follows:

### Step 1:

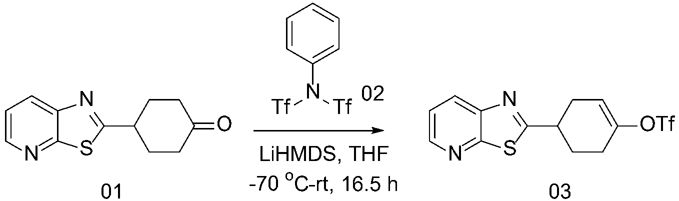

At room temperature, starting material **01** (900 mg, 3.88 mmol) was dissolved in anhydrous tetrahydrofuran (10 mL). Lithium bis(trimethylsilyl)amide (5.81 mL, 5.81 mmol, a solution of 1 M in tetrahydrofuran) was added dropwise at -70°C. The reaction mixture was reacted with stirring at -70°C for 0.5 hours, and then compound **02** (2.07 g, 5.79 mmol) was added. The reaction mixture was stirred at room temperature for 16.5 hours. Water (100 mL) was added, and the mixture was extracted with ethyl acetate (50 mL × 3), washed with a saturated salt solution (100 mL), dried over anhydrous sodium sulfate, filtered, and spin-dried. The residue was purified by silica gel column chromatography (petroleum ether:ethyl acetate = 4:1 to 3:1) to obtain a white solid as the target product (800 mg, 57.2%). LC-MS: 365.2 [M+H]⁺.

### Step 2:

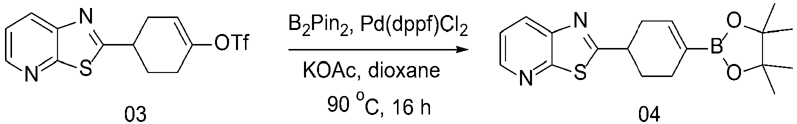

At room temperature, compound 03 (800 mg, 2.201 mmol), bis(pinacolato)diboron (840 mg, 3.307 mmol), [1,1'-bis(diphenylphosphino)ferrocene]dichloropalladium(II) (164 mg, 0.224 mmol), and potassium acetate (439 mg, 4.381 mmol) were dissolved in 1,4-dioxane (10 mL). The reaction mixture was reacted with stirring at 90°C under argon protection for 16 hours. Water (100 mL) was added, and the mixture was extracted with ethyl acetate (50 mL × 3), washed with a saturated salt solution (100 mL), dried over anhydrous sodium sulfate, filtered, and spin-dried. The residue was purified by silica gel column chromatography (petroleum ether:ethyl acetate = 3:1 to 2:1) to obtain a white solid as the target product (450 mg, 58.7%). LC-MS: 343.2 [M+H]⁺.

### Step 3:

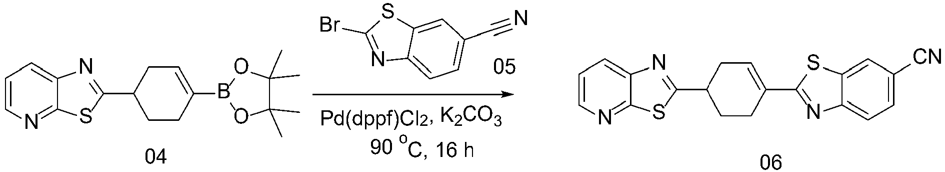

At room temperature, compound **04** (450 mg, 1.316 mmol), compound **05** (468 mg, 1.973 mmol), [1,1'-bis(diphenylphosphino)ferrocene]dichloropalladium(II) (97 mg, 0.131 mmol), and potassium carbonate (362 mg, 2.630 mmol) were dissolved in 1,4-dioxane (4 mL) and water (1 mL). The reaction mixture was reacted with stirring at 90°C under argon protection for 16 hours. Water (50 mL) was added, and the mixture was extracted with ethyl acetate (20 mL × 3), washed with a saturated salt solution (100 mL), dried over anhydrous sodium sulfate, filtered, and spin-dried. The residue was purified by silica gel column chromatography (ethyl acetate:petroleum ether = 33%) to obtain a brown solid as the target product (300 mg, 69.6%). LC-MS: 375.2 [M+H]⁺.

### Step 4:

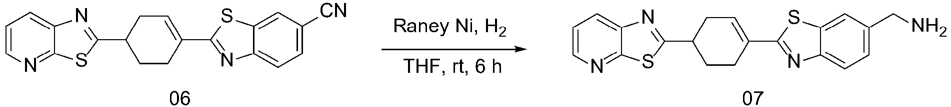

At room temperature, compound **06** (130 mg, 0.348 mmol) was dissolved in tetrahydrofuran (10 mL). Raney nickel (100 mg) was added at room temperature, and the reaction mixture was reacted with stirring at room temperature under a hydrogen balloon for 6 hours. After filtration and spin-drying, a colorless viscous liquid was obtained as the target product (80 mg, crude product). LC-MS: 379.1 [M+H]⁺.

### Step 5:

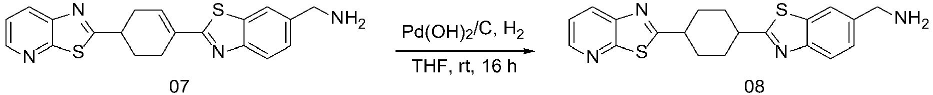

At room temperature, compound **07** (75 mg, 0.198 mmol) was dissolved in tetrahydrofuran (10 mL). Wet palladium hydroxide on carbon (100 mg) was added. After purging with hydrogen twice, the reaction mixture was reacted with stirring at room temperature under a hydrogen balloon for 16 hours. After filtration, the filtrate was spin-dried. The residue was separated by silica gel column chromatography (petroleum ether:ethyl acetate = 1:1 to 2:3) to obtain a yellow solid as the target product (20 mg, yield: 26.7%). LC-MS: 381.2 [M+H]⁺.

### Step 6:

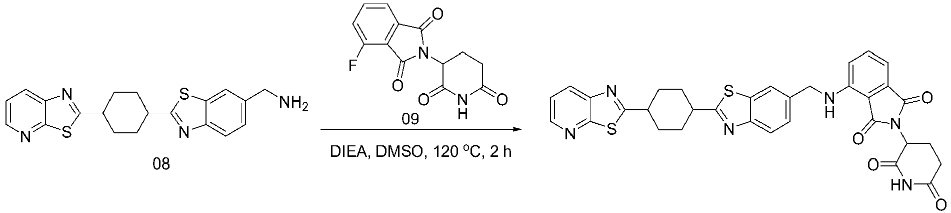

At room temperature, compound **08** (15 mg, 0.0395 mmol), compound **09** (15 mg, 0.0543 mmol), and *N*,*N*-diisopropylethylamine (15 mg, 0.116 mmol) were dissolved in dimethylsulfoxide (1 mL). The reaction mixture was reacted with stirring at 120°C for 2 hours. The mixture was cooled to 20°C, and then separated by preparative liquid chromatography (C18, 5-100% acetonitrile/water as a mobile phase, and 0.1% formic acid) to obtain the title compound, a yellow solid (2.17 mg, yield: 20.9%). LC-MS: 637.4 [M+H]⁺. ¹H NMR (400 MHz, DMSO-*d*₆) δ11.10 (s, 1H), 8.56 (d, *J =* 4.3 Hz, 1H), 8.32 (d, *J =* 7.9 Hz, 1H), 8.03 (s, 1H), 7.90 (d, *J =* 8.4 Hz, 1H), 7.56 - 7.46(m, 3H), 7.33 (s, 1H), 7.00 (dd, *J* = 18.0, 7.8 Hz, 2H), 5.08 (dd, *J =* 12.5, 5.1 Hz, 1H), 4.69 (d, *J* = 5.7 Hz, 2H), 3.45 (s, 1H), 2.96 - 2.85 (m, 1H), 2.60 (d, *J =* 16.1 Hz, 2H), 2.20 - 2.01 (m, 10H).

### Example 7: Preparation of Compound 7

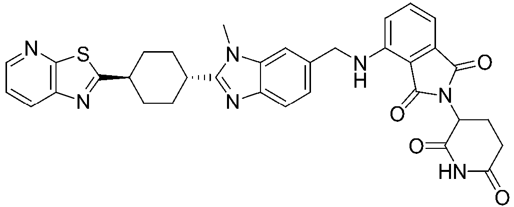

The synthesis steps were as follows:

### Step 1:

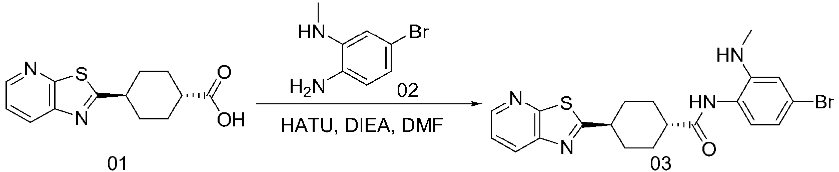

Compound **01** (719 mg, 2.74 mmol), compound **02** (550 mg, 2.74 mmol), and *N,N-*diisopropylethylamine (1.06 g, 8.22 mmol) were dissolved in anhydrous N,N-dimethylformamide (12 mL). (7-Azobenzotriazole)-tetramethylurea hexafluorophosphate (1.56 g, 4.11 mmol) was added with stirring at room temperature. The reaction mixture was reacted with stirring at room temperature for 2 hours. The mixture was added with water (30 mL), and filtered to obtain a red solid as the target product (990 mg, yield: 81.2%). LC-MS: 445.1, 447.1 [M+H]⁺

### Step 2:

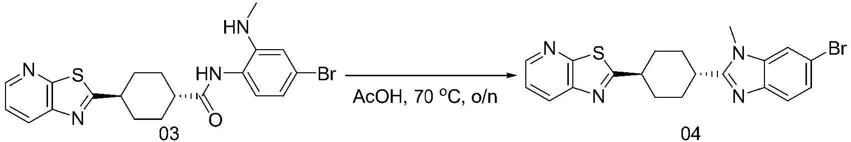

Compound **03** (990 mg, 2.22 mmol) was dissolved in acetic acid (10 mL). The reaction mixture was reacted with stirring at 70°C for 1 hour. The mixture was spin-dried to obtain a purple solid as the target product (700 mg, yield: 73.6%). LC-MS: 427.0, 429.0 [M+H]⁺.

### Step 3:

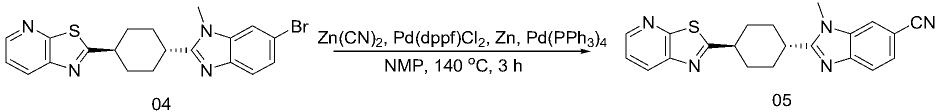

At room temperature, compound **04** (540 mg, 1.26 mmol), zinc cyanide (296 mg, 2.52 mmol), zinc powder (16.5 mg, 0.252 mmol), [1,1'-bis(diphenylphosphino)ferrocene]palladium dichloride-dichloromethane complex (204 mg, 0.252 mmol), tetrakis(triphenylphosphine)palladium (291 mg, 0.252 mmol), and anhydrous N-methylpyrrolidone (20 mL) were added to a sealed tube. The reaction mixture was reacted with stirring at 140°C for 3 hours. After filtration, the filtrate was added with water (100 mL), extracted with ethyl acetate (20 mL × 3), washed with a saturated salt solution (50 mL), dried over anhydrous sodium sulfate, filtered, and spin-dried. The residue was slurried (PE:EA = 1:1, 20 mL) to obtain a white solid as the target product (349 mg, yield: 73.9%). LC-MS: 374.1 [M+H]⁺.

### Step 4:

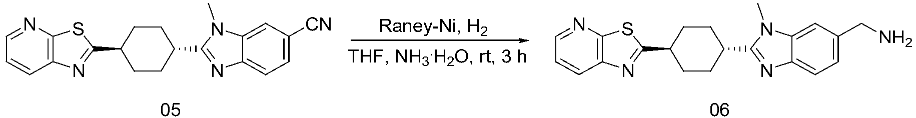

Compound **05** (340 mg, 0.910 mmol) was dissolved in tetrahydrofuran (15 mL). Raney nickel (100 mg) was then added, and 2 drops of aqueous ammonia were added dropwise. The reaction mixture was reacted with stirring at room temperature under a hydrogen atmosphere for 3 hours. After filtration, the filtrate was spin-dried to obtain a red solid as the target product (300 mg, yield: 87.3%). LC-MS: 378.1 [M+H]⁺.

### Step 5:

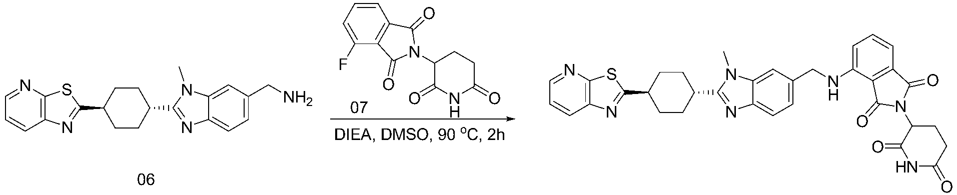

Compound **06** (170 mg, 0.450 mmol), compound **07** (124 mg, 0.450 mmol), and *N,N-*diisopropylethylamine (174 mg, 1.35 mmol) were dissolved in anhydrous dimethylsulfoxide (4 mL). The reaction mixture was reacted with stirring at 90 °C for 2 hours. The mixture was separated by preparative liquid chromatography (C18, 5-100% acetonitrile/water as a mobile phase, 0.03% trifluoroacetic acid) twice to obtain the title compound, a yellow-green solid (48.35 mg, yield: 16.9%). LC-MS: 634.3 [M+H]⁺, ¹H NMR (400 MHz, DMSO-*d*₆) δ 11.11 (s, 1H), 8.60 (dd, *J* = 4.6, 1.3 Hz, 1H), 8.36 (dd, *J =* 8.2, 1.3 Hz, 1H), 7.96 (s, 1H), 7.77 (dd, *J* = 8.4, 1.8 Hz, 1H), 7.63 - 7.56 (m, 2H), 7.53 - 7.47 (m, 1H), 7.37 (t, *J* = 5.8 Hz, 1H), 7.04 (d, *J =* 7.1 Hz, 1H), 7.01 (d, *J* = 8.6 Hz, 1H), 5.08 (dd, *J* = 12.9, 5.4 Hz, 1H), 4.75 (d, *J* = 5.9 Hz, 2H), 4.00 (s, 3H), 3.55 - 3.47 (m, 1H), 3.34 - 3.27 (m, 1H), 2.95 - 2.85 (m, 1H), 2.64 - 2.52 (m, 2H), 2.40 - 2.31 (m, 2H), 2.28 - 2.19 (m, 2H), 2.09 - 2.01 (m, 1H), 1.96 - 1.82 (m, 4H).

### Activity Test Example:

### Example 1: DC50 Test of Compounds

### 1. Experimental materials

The IKZF1/Jurkat HiBiT cell line was constructed by Shanghai Runnuo Biotechnology Co., Ltd. RPMI-1640 medium was purchased from Gibco (Cat # C11875500BT), FBS from Gibco (Cat #10091148), Nano Glo HiBiT Lytic Detection System from Promega (Cat #N3040), and 96-well plates from Corning (Cat #3610).

### 2. Experimental method

The degradation effect of compounds on IKZF1 was determined by measuring the HiBiT signal intensity in cells.

First, the cell suspension was collected and centrifuged, and the supernatant was discarded. The separated cells by centrifugation were resuspended in a fresh complete medium and the cell density was adjusted to 2.5*10⁵ cells/mL. The resuspended cells were seeded into an opaque 96-well cell culture plate at 100 µL/well, and the plate was placed in a 37°C cell culture incubator for later use. Then, diluted compounds at different concentrations were added by 1 ul to the wells, and the plate was incubated in the cell culture incubator for 6 hours. After 6 hours, 100 µL of Nano Glo HiBiT Lytic Detection System reagent was added, and the luminescence values were read on a plate reader.

### 3. Data Analysis

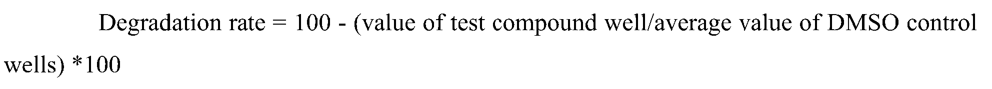

Degradation rate = 100 - (value of test compound well/average value of DMSO control wells) *100

Using the degradation rate corresponding to each gradient concentration well and the respective concentration, a gradient curve of degradation rates was fitted using GraphPad software, and the DC50 values of the compounds were calculated.

### 4. Experimental Results

According to the experimental method described above, the obtained DC50 values of the compounds are shown in the table below.

**Table 1 Detection Results**

| Compound No. | Compound Structure | HiBiT DC50/nM IKZF1 |
|---|---|---|
| 1 | 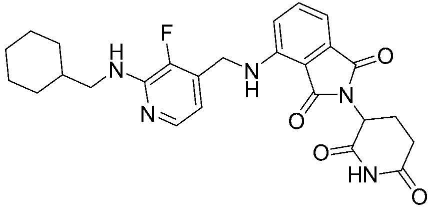 | <10 |
| 2 | 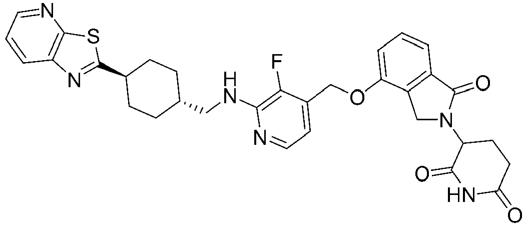 | <10 |
| 3 | 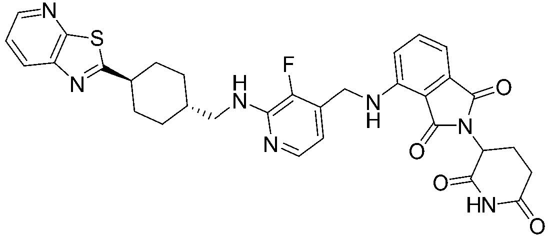 | <10 |
| 4 | 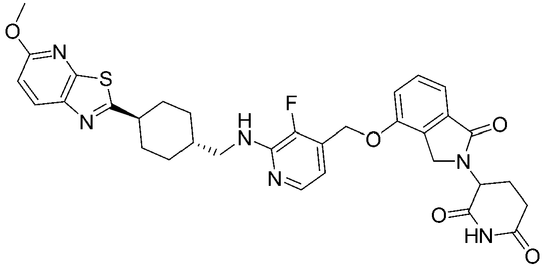 | <10 |
| 5 | 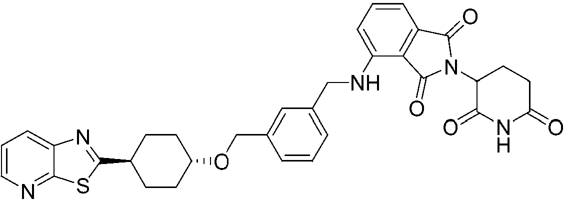 | 50<DC50<50 0 |
| 6 | 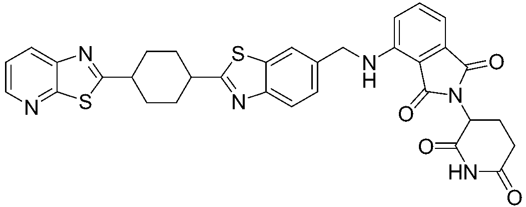 | <10 |
| 7 | 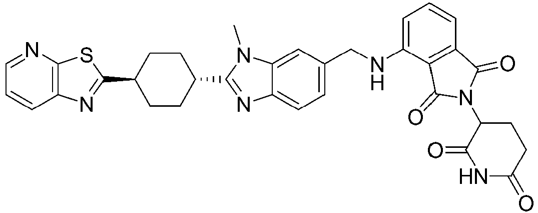 | <10 |

The foregoing descriptions are merely preferred embodiments of the present disclosure, and are not intended to limit the present disclosure. Any modifications, equivalent substitutions, and the like made within the spirit and principles of the present disclosure shall fall within the protection scope of the present disclosure.

The foregoing embodiments and methods described in the present disclosure may vary based on the abilities, experience, and preferences of those skilled in the art.

The method steps listed only in a particular order in the present disclosure do not constitute any limitation on the order of the method steps.

## Claims

1. A compound, or a pharmaceutically acceptable salt, stereoisomer, ester, prodrug, solvate, and deuterated compound thereof, the compound having the following structure: 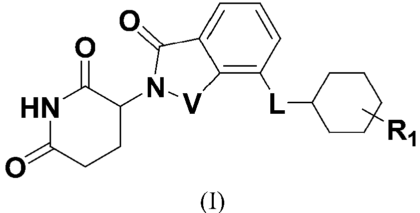 wherein
V is selected from the group consisting of: -CH₂- and 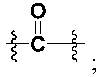
L has the following structure: 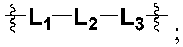
L₁ is selected from the group consisting of: -NH-(C₀₋₁₀ alkylene)- and -O-(C₀₋₁₀ alkylene)-;
L₂ is selected from the group consisting of: 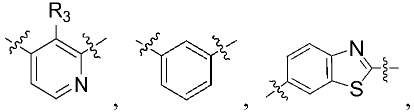
and 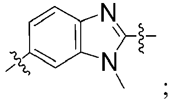 R₃ is selected from the group consisting of: -H, halogen, -CN, -NO₂, -CF₃, -CHF₂, -CH₂F, -OH, -OCH₃, -OCF₃, -OCHF₂, -OCH₂F, and C₁₋₃ alkyl;
L₃ is selected from the group consisting of: -(C₀₋₁₀ alkylene)-, -(C₀₋₆ alkylene)-NH-(C₀₋₆ alkylene)-, and -(C₀₋₆ alkylene)-O-(C₀₋₆ alkylene)-;
R₁ is one or more independent substituents on a ring 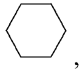 and is selected from the group consisting of: =O, halogen, -CN, -NO₂, -CF₃, -OCF₃, C₀₋₁₀ alkyl, -O(C₀₋₁₀ alkyl), -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -SC₀₋₁₀ alkyl, -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), and 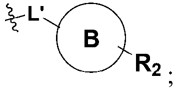 L' is selected from the group consisting of: a single bond, -(C₀₋₁₀ alkylene)-, - (C₀₋₁₀ alkylene)-NR^{L1}-( C₀₋₁₀ alkylene)-, -(C₀₋₁₀ alkylene)-O-(C₀₋₁₀ alkylene)-, -(C₀₋₁₀ alkylene)-S-(C₀₋₁₀ alkylene)-, -(C₀₋₁₀ alkylene)-NR^{L1}CO-(C₀₋₁₀ alkylene)-, -(C₀₋₁₀ alkylene)-CONR^{L1}-(C₀₋₁₀ alkylene)-, -(C₀₋₁₀ alkylene)-CO-(C₀₋₁₀ alkylene)-, -(C₀₋₁₀ alkylene)-SO₂-(C₀₋₁₀ alkylene)-, and - (C₀₋₁₀ alkylene)-SO-(C₀₋₁₀ alkylene)-; R^{L1} is selected from the group consisting of: H, C₁₋₆ alkyl, C₃₋₆ cycloalkyl, and 4- to 10-membered heterocycloalkyl; and R₂ is one or more independent substituents on a ring B, and is selected from the group consisting of: =O, a halogen, -CN, -NO₂, -CF₃, -OCF₃, C₀₋₁₀ alkyl, -O(C₀₋₁₀ alkyl), -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -SC₀₋₁₀ alkyl, -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), - CON(C₀₋₁₀ alkyl)(C₀₋₁₀ alkyl), -CO(C₀₋₁₀ alkyl), -(C₀₋₁₀ alkylene)-(C₃₋₆ cycloalkyl), -(C₀₋₁₀ alkylene)-(C₆₋₁₀ aryl), -(C₀₋₁₀ alkylene)-(4- to 10-membered heterocycloalkyl), and -(C₀₋₁₀ alkylene)-(4- to 10-membered heterocycloaryl), wherein one or more H atoms in the alkylene, alkyl, cycloalkyl, aryl, heterocycloalkyl, or heterocycloaryl are optionally substituted by a group selected from the group consisting of: =O, a halogen, -CN, -NO₂, -CF₃, -OCF₃, C₀₋₁₀ alkyl, -O(C₀₋₁₀ alkyl), -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -SC₀₋₁₀ alkyl, -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), - CO(C₀₋₁₀ alkyl), -(C₀₋₁₀ alkylene)-(C₃₋₆ cycloalkyl), -(C₀₋₁₀ alkylene)-(C₆₋₁₀ aryl), -(C₀₋₁₀ alkylene)-(4- to 10-membered heterocycloalkyl), and -(C₀₋₁₀ alkylene)-(4- to 10-membered heterocycloaryl).

2. The compound according to claim 1, **characterized in that** L₁ is selected from the group consisting of: -NH-, -NH-CH₂-, -NH-CH₂CH₂-, -NH-CH₂CH₂CH₂-, -O-, -O-CH₂-, -O-CH₂CH₂-, and -O-CH₂CH₂CH₂-.

3. The compound according to any one of claims 1 to 2, **characterized in that** L₃ is selected from the group consisting of: -NH-, -CH₂-NH-, -CH₂CH₂-NH-, -CH₂CH₂CH₂-NH-, -O-, -CH₂-O-, -CH₂CH₂-O-, -CH₂CH₂CH₂-O-, a single bond, -CH₂-, -CH₂CH₂-, -CH₂CH₂CH₂-, -NH-CH₂-, -NH-CH₂CH₂-, -NH-CH₂CH₂CH₂-, -O-CH₂-, -O-CH₂CH₂-, and -O-CH₂CH₂CH₂-.

4. The compound according to any one of claims 1 to 3, **characterized in that** L is selected from the following structures:

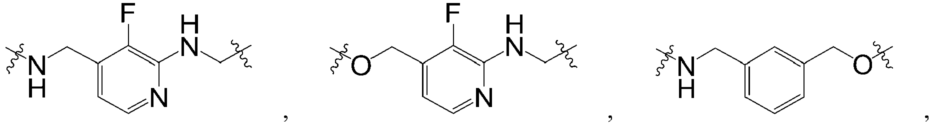

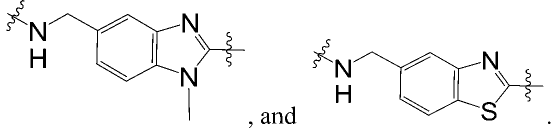

5. The compound according to any one of claims 1 to 4, **characterized in that** R₁ is selected from the group consisting of: -H, a halogen, -OH, C₁₋₆ alkyl, and -O(C₁₋₆ alkyl).

6. The compound according to any one of claims 1 to 5, **characterized in that** a moiety 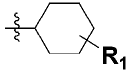 is

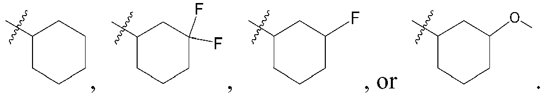

7. The compound according to any one of claims 1 to 4, **characterized in that** the compound has the following structure: 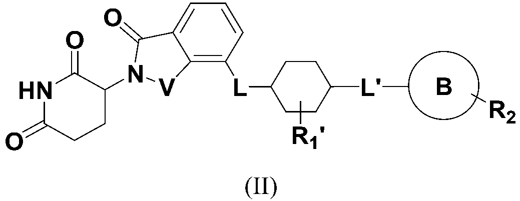 wherein R₁' is one or more independent substituents on a ring 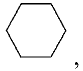 and is selected from the group consisting of: H, a halogen, -CN, -NO₂, -CF₃, -OCF₃, C₁₋₁₀ alkyl, -O(C₀₋₁₀ alkyl), -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -SC₀₋₁₀ alkyl, -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl).

8. The compound according to claim 7, **characterized in that** the ring B is a benzene ring, a bicyclic aromatic ring, a tricyclic aromatic ring, a monocyclic heteroaromatic ring, a bicyclic heteroaromatic ring, or a tricyclic heteroaromatic ring.

9. The compound according to claim 8, **characterized in that** the ring B is selected from the group consisting of:

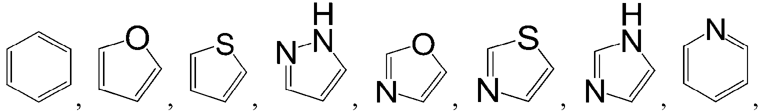

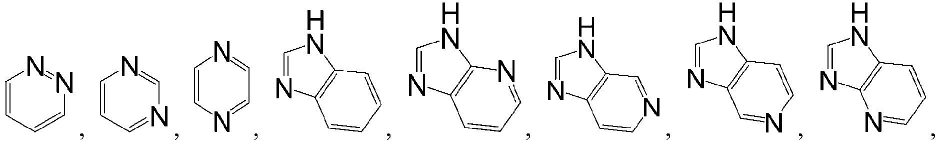

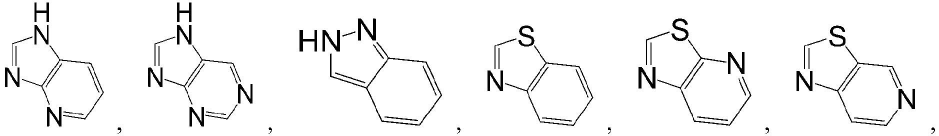

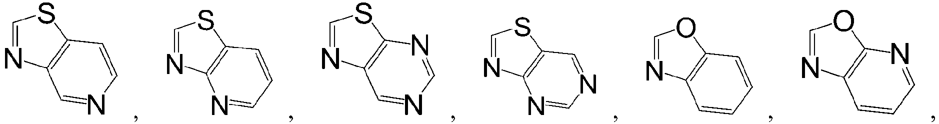

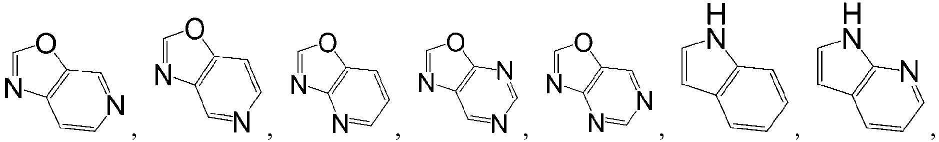

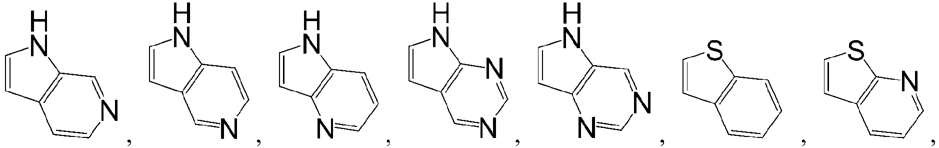

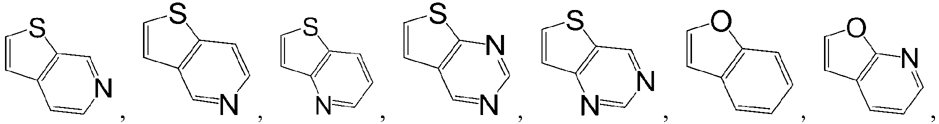

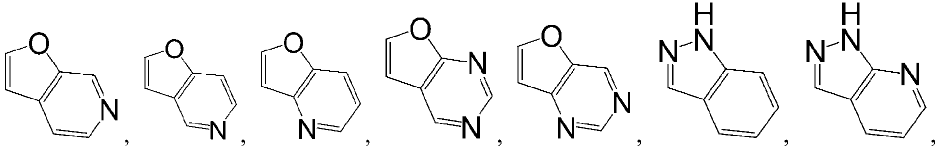

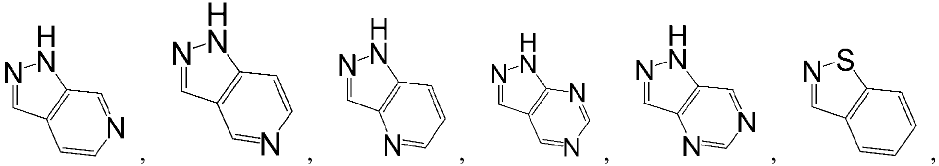

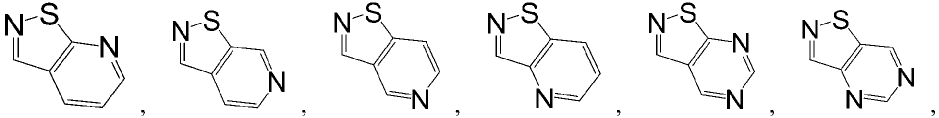

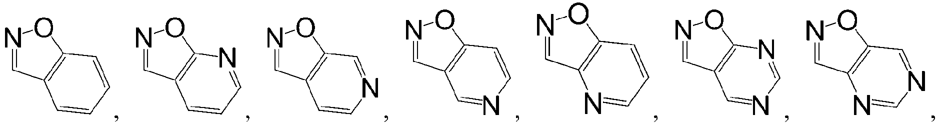

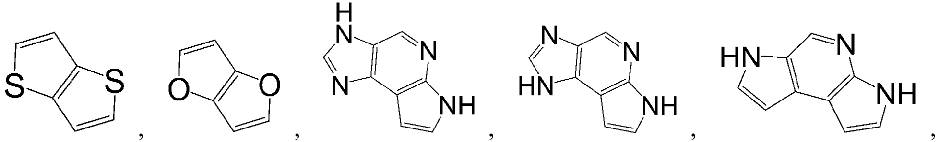

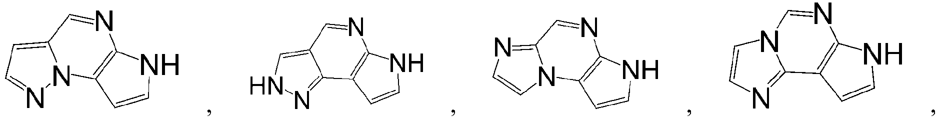

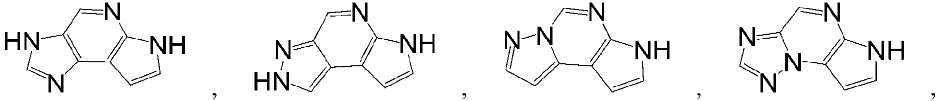

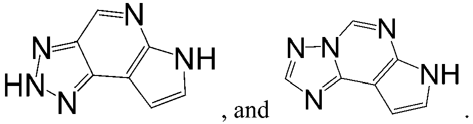

10. The compound according to claim 7 or 8, **characterized in that** R₂ is selected from the group consisting of: -H, -F, -Cl, -Br, -I, -CF₃, -CHF₂, -CH₂F, -CH₂CF₃, -CN, -NO₂, -CH₃, 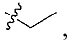 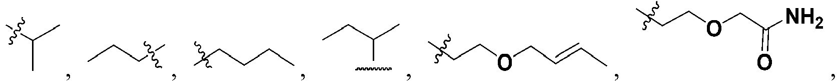 -OH, 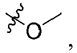 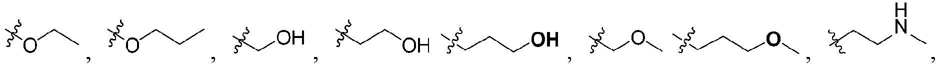 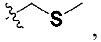 -NH₂, 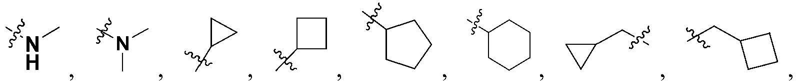 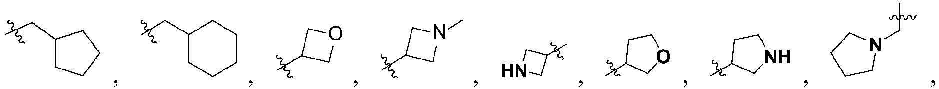 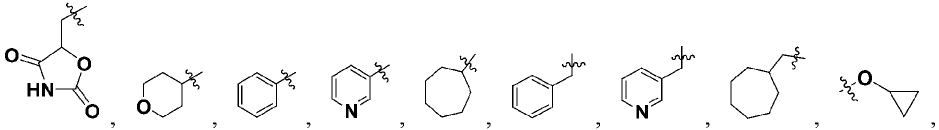 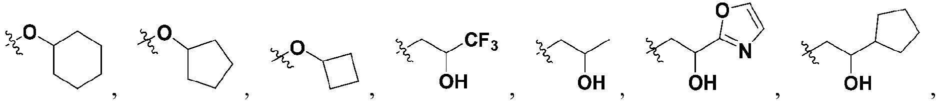 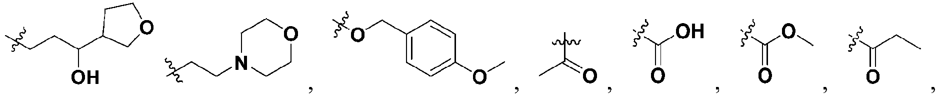 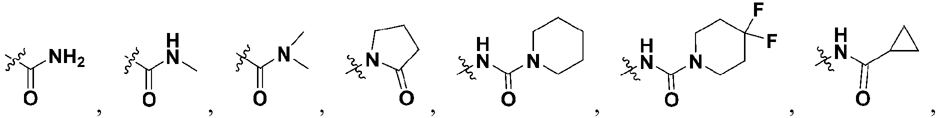 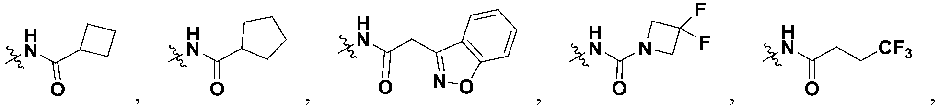 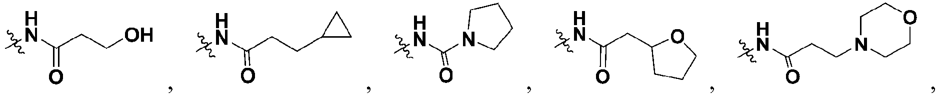 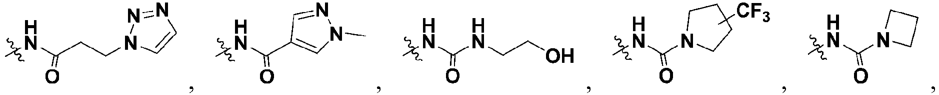 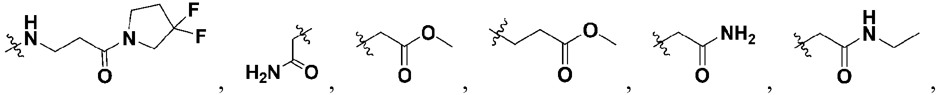 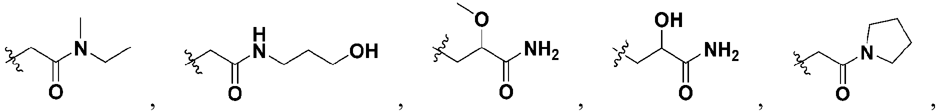 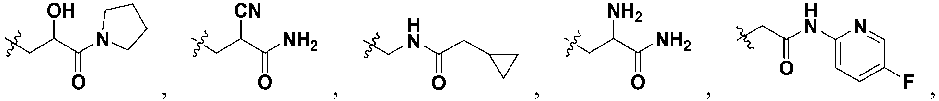 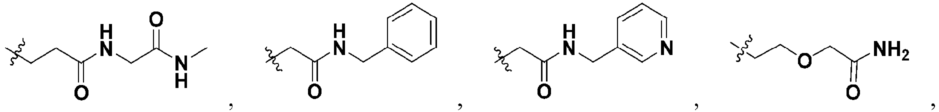 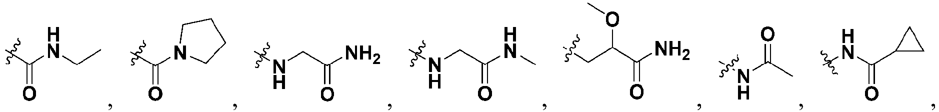 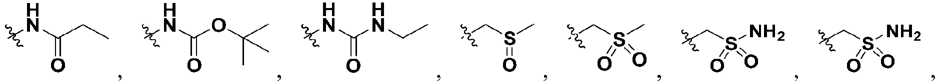 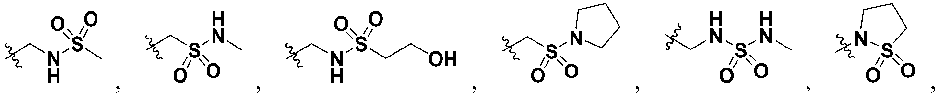 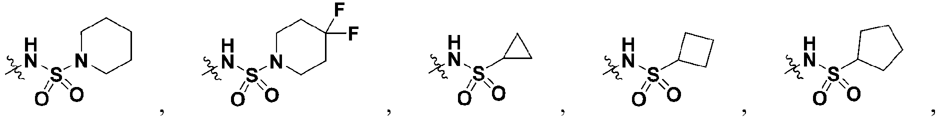 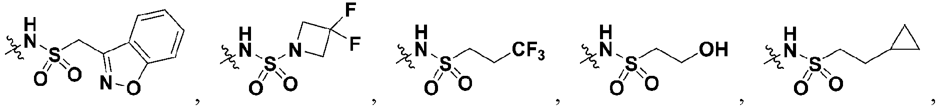 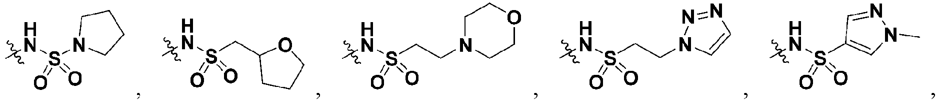 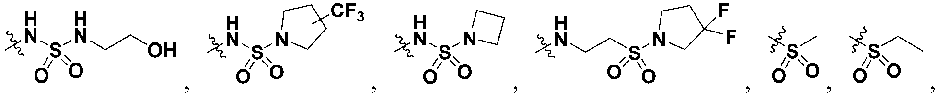 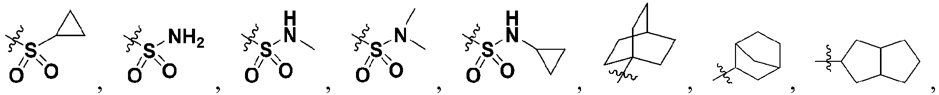 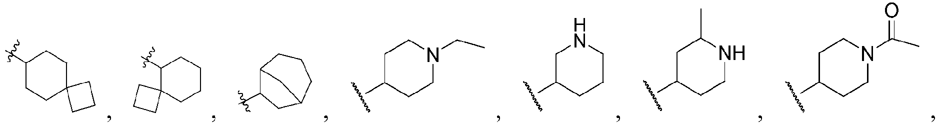 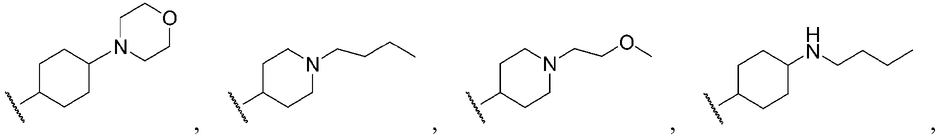 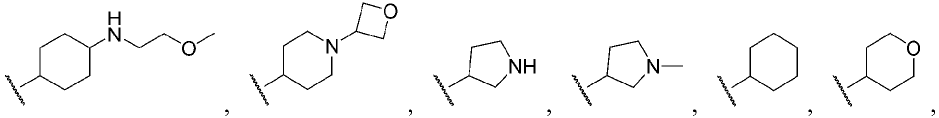 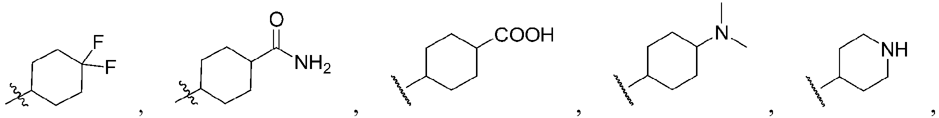 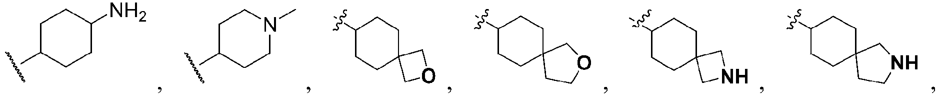 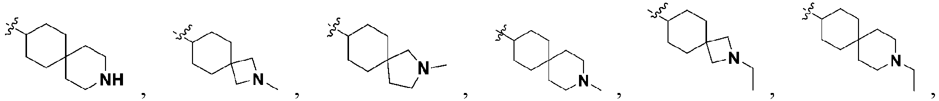 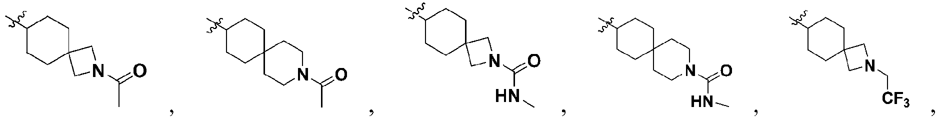 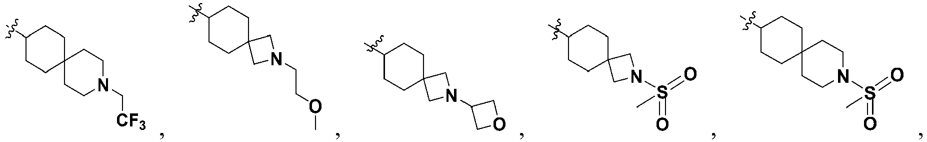 and

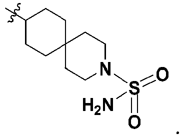

11. The compound according to claim 10, **characterized in that** R₂ is selected from the group consisting of: -H, -F, -Cl, -Br, -I, -CF₃, -CHF₂, -CH₂F, -CH₂CF₃, -CN, -NO₂, -CH₃,

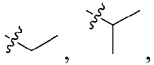

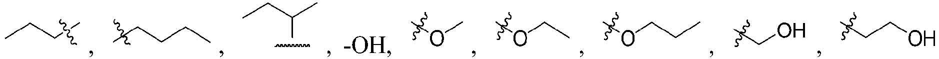

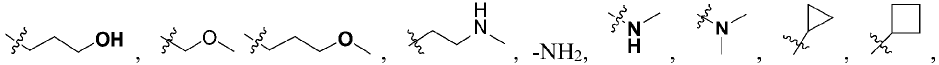

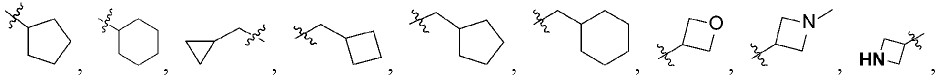

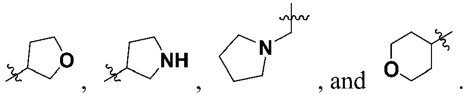

12. The compound according to claim 7, **characterized in that** a moiety 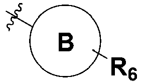 is selected from the following structures:

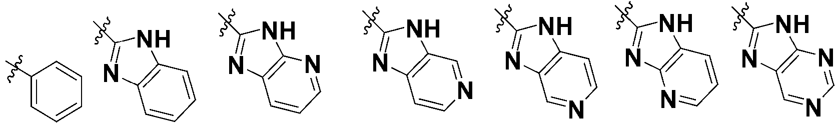

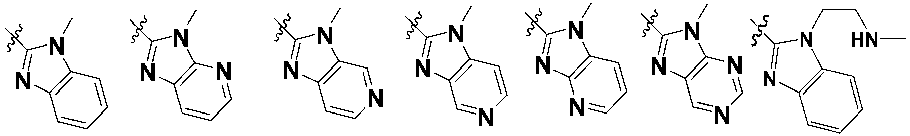

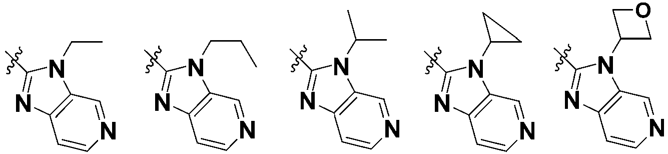

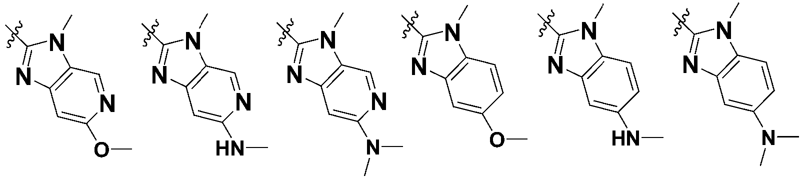

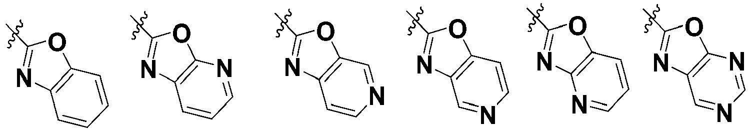

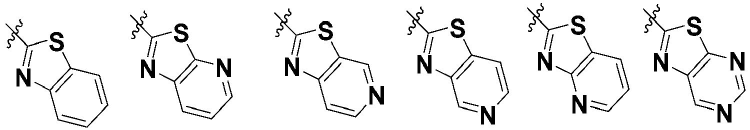

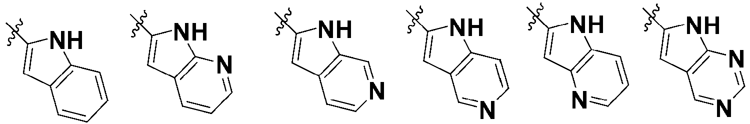

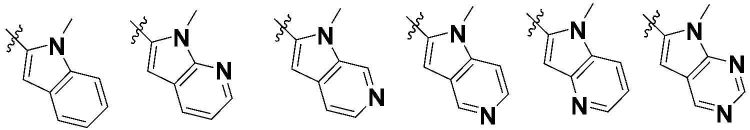

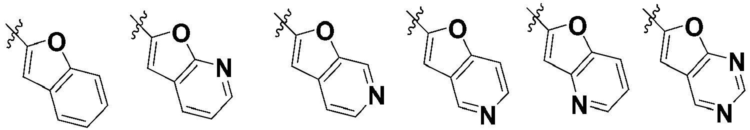

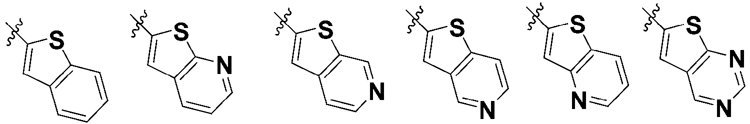

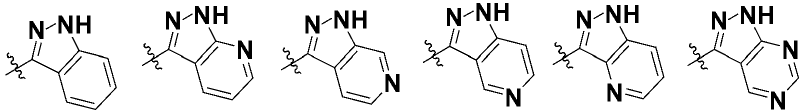

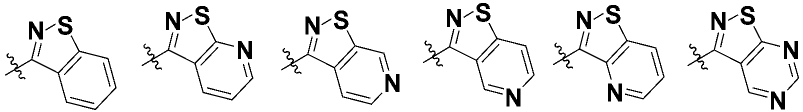

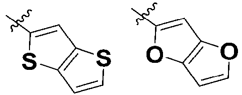

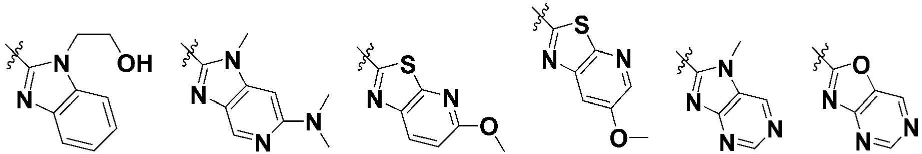

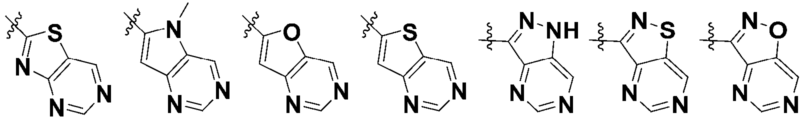

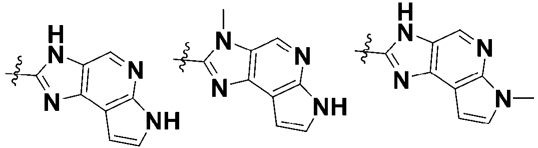

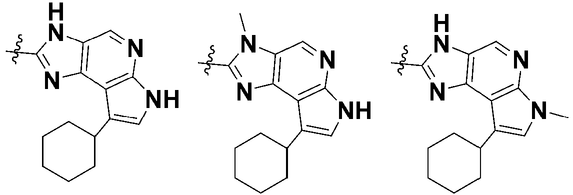

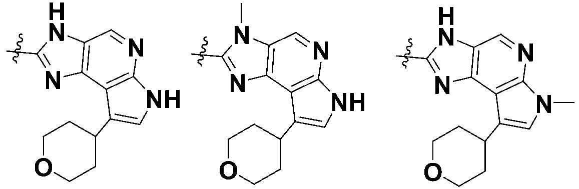

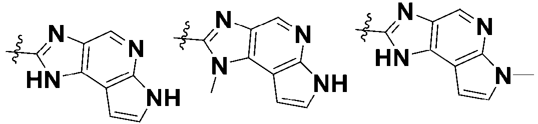

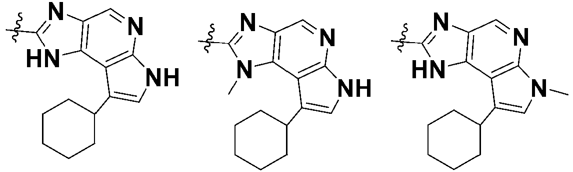

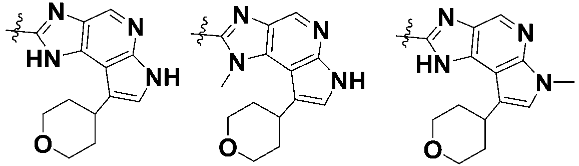

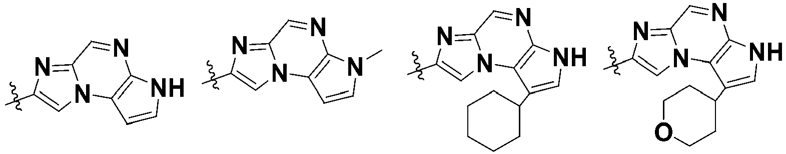

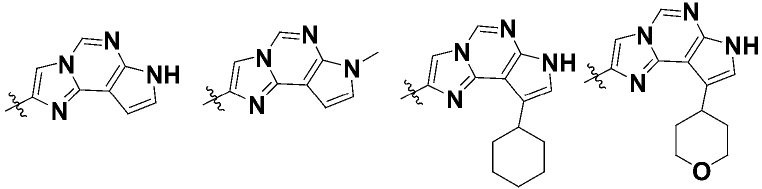

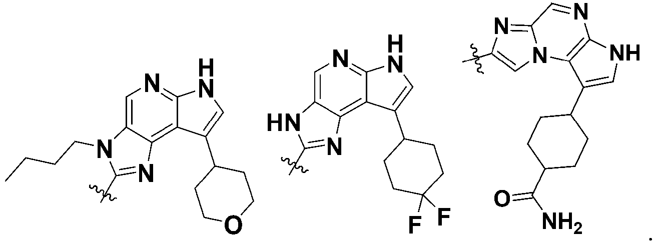

13. The compound according to claim 1, **characterized in that** the compound is selected from the following structures:

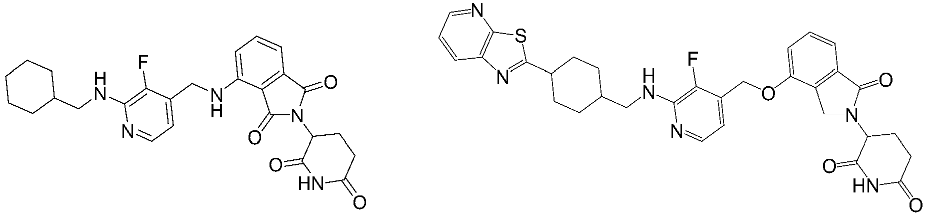

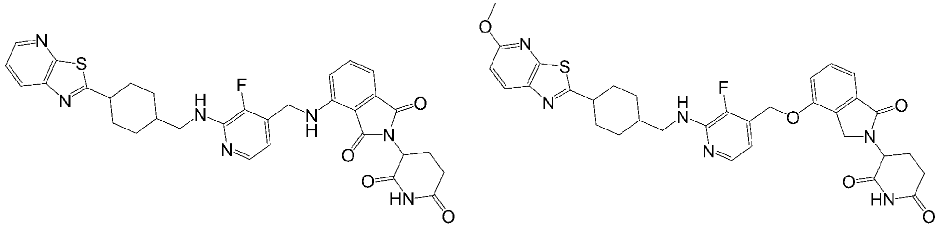

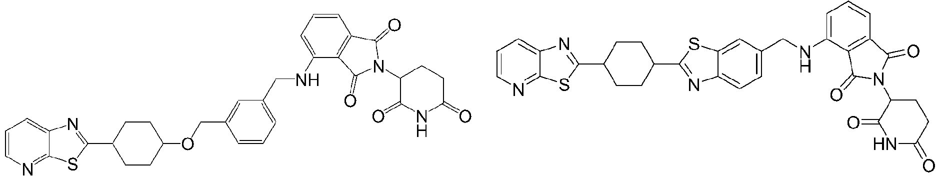

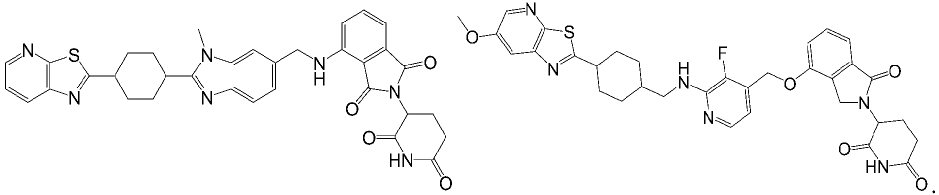

14. The compound according to claim 1, **characterized in that** the stereoisomer is selected from the following structure:

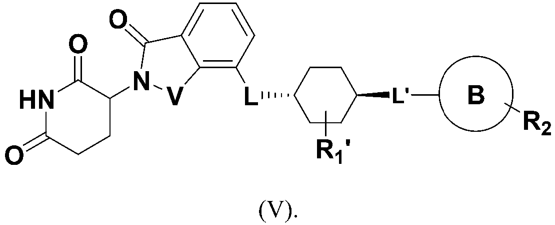

15. The compound according to claim 14, **characterized in that** the stereoisomer is selected from the following structures:

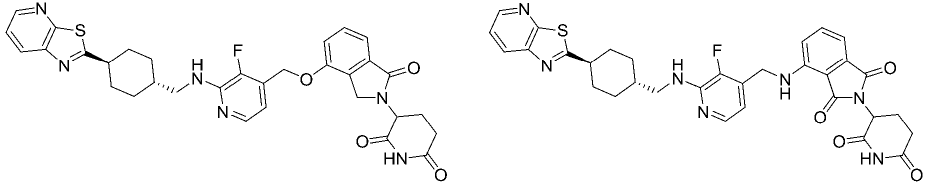

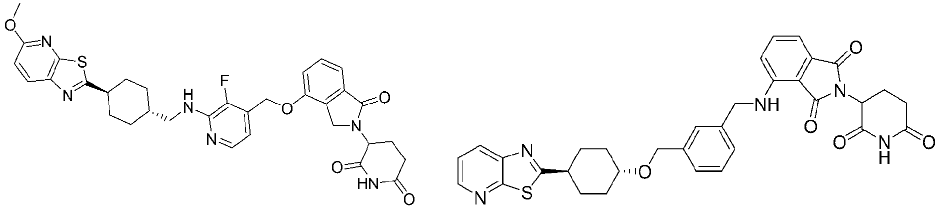

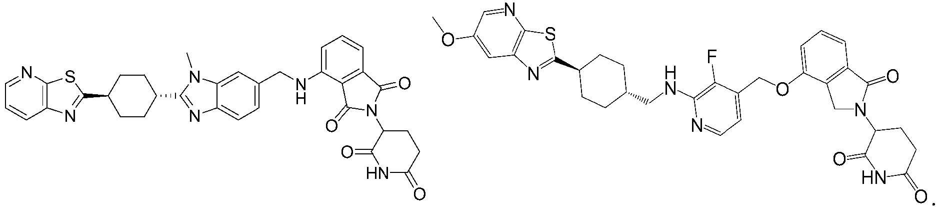

16. A pharmaceutical composition, **characterized by** comprising the compound according to any one of claims 1 to 15, or a pharmaceutically acceptable salt, stereoisomer, ester, prodrug, solvate, and deuterated compound thereof, and one or more pharmaceutically acceptable excipients.

17. Use of the compound according to any one of claims 1 to 5, or a pharmaceutically acceptable salt, stereoisomer, ester, prodrug, solvate, and deuterated compound thereof, in preparing a medicament for preventing and/or treating an IKZF1-related disease,
the disease being selected from the group consisting of: a tumor, an autoimmune disease, an inflammatory disease, a disease related to pathogen infection, a neurodegenerative disease, and a xenoimmune disease.

18. The use according to claim 17, **characterized in that** the tumor is selected from the group consisting of: adrenal carcinoma, anal carcinoma, angiosarcoma, appendiceal carcinoma, biliary tract carcinoma, bladder carcinoma, breast carcinoma, brain carcinoma, bronchial carcinoma, carcinoid tumor, cervical carcinoma, choriocarcinoma, chordoma, craniopharyngioma, colorectal carcinoma, connective tissue carcinoma, epithelial carcinoma, ependymoma, endotheliosarcoma, endometrial carcinoma, esophageal carcinoma, Ewing's sarcoma, ocular carcinoma, gallbladder carcinoma, gastric carcinoma, gastrointestinal stromal tumor (GIST), germ cell carcinoma, head and neck carcinoma, hematological malignancy, hemangioblastoma, hypopharyngeal carcinoma, inflammatory myofibroblastic tumor, immunocytic amyloidosis, kidney carcinoma, liver carcinoma, lung carcinoma, leiomyosarcoma (LMS), muscle carcinoma, mesothelioma, myeloproliferative disorder (MPD), neuroblastoma, neurofibroma, neuroendocrine carcinoma, osteosarcoma, ovarian carcinoma, papillary adenocarcinoma, pancreatic carcinoma, penile carcinoma, pinealoma, primitive neuroectodermal tumor (PNT), prostate carcinoma, rhabdomyosarcoma, salivary gland carcinoma, skin carcinoma, small intestinal carcinoma, soft tissue sarcoma, sebaceous gland carcinoma, sweat gland carcinoma, synovioma, testicular carcinoma, thyroid carcinoma, urethral carcinoma, vaginal carcinoma, and vulvar carcinoma;
wherein the hematological malignancy is selected from the group consisting of: leukemia, a lymphoma, and multiple myeloma;
wherein the leukemia is chronic lymphocytic leukemia, chronic myeloid leukemia, acute lymphoblastic leukemia, acute myeloid leukemia, or acute monocytic leukemia; and
wherein the lymphoma is B-cell lymphoma, T-cell lymphoma, or NK/T-cell lymphoma.

19. The use according to claim 17, **characterized in that** the autoimmune disease is selected from the group consisting of: type I diabetes mellitus, systemic lupus erythematosus, systemic vasculitis, regional enteritis, cardiomyopathy, hemolytic anemia, fibromyalgia, Graves' disease, ulcerative colitis, Crohn's disease, multiple sclerosis, myasthenia gravis, myositis, neutropenia, psoriasis, chronic fatigue syndrome, juvenile arthritis, juvenile diabetes, scleroderma, psoriatic arthritis, Sjögren's syndrome, rheumatic fever, rheumatoid arthritis, sarcoidosis, idiopathic thrombocytopenic purpura (ITP), Hashimoto's disease, mixed connective tissue disease, interstitial cystitis, pernicious anemia, leukoencephalitis, alopecia areata, ankylosing spondylitis, primary biliary cirrhosis, anti-Glomerular Basement Membrane (GBM) nephritis, anti-Tubular Basement Membrane (TBM) nephritis, antiphospholipid syndrome, polymyalgia rheumatica, polymyositis, autoimmune Addison's disease, chronic active hepatitis, vitiligo, psoriasis, autoimmune hyperlipidemia, autoimmune myocarditis, temporal arteritis, autoimmune thyroid disease, axonal and neuronal neuropathies, Behçet's disease, bullous pemphigoid, allergic asthma, osteoarthritis, Chagas' disease, uveitis, chronic inflammatory demyelinating polyneuropathy (CIDP), cicatricial pemphigoid/benign mucosal pemphigoid, Cogan's syndrome, congenital heart block, Coxsackie viral myocarditis, demyelinating neuropathy, dermatomyositis, discoid lupus, lens-induced uveitis, polyarteritis nodosa, Dressler's syndrome, idiopathic mixed cryoglobulinemia, Evans syndrome, Goodpasture syndrome, allergic rhinitis, infectious neuronitis, hypogammaglobulinemia, inclusion body myositis, vesiculobullous skin disease, Wegener's granulomatosis, Ménière's disease, Lambert-Eaton syndrome, Mooren's ulcer, atypical celiac disease, ocular cicatricial pemphigoid, pemphigus vulgaris, perivenous encephalomyelitis, postpericardiotomy syndrome, scleritis, sperm testicular autoimmunity, stiff-person syndrome, subacute bacterial endocarditis (SBE), sympathetic ophthalmia, transverse myelitis and necrotizing myelopathy, type I autoimmune polyglandular syndrome, type II autoimmune polyglandular syndrome, pernicious anemia, and endometriosis; and
wherein the autoimmune disease is systemic lupus erythematosus, type I diabetes mellitus, or autoimmune thyroid disease.
